(19) Europäisches Patentamt
European Patent Office
Office européen des brevets

(11) **EP 4 213 738 B1**

(12) **EUROPEAN PATENT SPECIFICATION**

(45) Date of publication and mention
of the grant of the patent:
**06.11.2024 Bulletin 2024/45**

(21) Application number: **21773039.9**

(22) Date of filing: **01.09.2021**

(51) International Patent Classification (IPC):
*A61B 7/00* *(2006.01)* *A61B 7/04* *(2006.01)*

(52) Cooperative Patent Classification (CPC):
**A61B 7/003; A61B 7/04**

(86) International application number:
**PCT/EP2021/074176**

(87) International publication number:
**WO 2022/058169 (24.03.2022 Gazette 2022/12)**

(54) **A WEARABLE DEVICE AND A METHOD OF USING A WEARABLE DEVICE**

WEARABLE-VORRICHTUNG UND VERFAHREN ZUR VERWENDUNG EINER
WEARABLE-VORRICHTUNG

DISPOSITIF POUVANT ÊTRE PORTÉ ET PROCÉDÉ D'UTILISATION D'UN DISPOSITIF POUVANT
ÊTRE PORTÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.09.2020 US 202063080328 P**

(43) Date of publication of application:
**26.07.2023 Bulletin 2023/30**

(73) Proprietor: **Koninklijke Philips N.V.
5656 AG Eindhoven (NL)**

(72) Inventors:
 • **DELLIMORE, Kiran, Hamilton, J.
 5656 AE Eindhoven (NL)**
 • **BRANS, Harold, Johannes, Antonius
 5656 AE Eindhoven (NL)**
 • **DEN BRINKER, Albertus, Cornelis
 5656 AE Eindhoven (NL)**
 • **OUWELTJES, Okke
 5656 AE Eindhoven (NL)**
 • **DE GRAAF, Pascal
 5656 AE Eindhoven (NL)**
 • **BARAGONA, Marco
 5656 AE Eindhoven (NL)**
 • **BOU JAWDE, Samer
 5656 AE Eindhoven (NL)**

(74) Representative: **Philips Intellectual Property &
Standards
High Tech Campus 52
5656 AG Eindhoven (NL)**

(56) References cited:
**US-A1- 2014 100 469    US-A1- 2014 276 150
US-A1- 2018 272 147    US-A1- 2018 338 721**

## Description

FIELD OF THE INVENTION

The invention relates to a wearable device according to claim 1 and a method of using a wearable device according to claim 7.

BACKGROUND OF THE INVENTION

Many patients with chronic respiratory diseases, such as chronic obstructive pulmonary disease (COPD) and cystic fibrosis (CF), experience severe mucus build-up in their lungs. They must periodically clear the mucus, which is often difficult to expel. Various methods are typically employed to first loosen and/or thin the mucus prior to expulsion by coughing. Loosening and/or thinning of the mucus is usually achieved by manual means (e.g. chest percussion) or semi-automated means (e.g. high frequency chest wall oscillation therapy or HFCWO). In the latter case, HFCWO device settings are currently not optimized to meet patient-specific mucus removal needs. For example, CF patients typically have very thick, viscous mucus, while COPD patients have an excess amount of mucus with viscosity in between normal and CF mucus viscosities, which are both very different from normal mucus viscosities.

These different mucus build-up situations require very different vest settings, often in combination with mucolytic medication, in order to ensure effective mucus loosening and/or thinning. However, commercially available HFCWO vests do not offer a means to quantify mucus properties and therefore are unable to deliver dynamic personalized therapy during a therapy session. To overcome this limitation, it has been proposed to perform lung sound analysis using microphones embedded in the HFCWO vest. It would be desirable to minimise attenuation of noise and acoustic interference during the lung sound acquisition process in order to improve signal quality.

US 2019/0142686 describes a wearable device configured to oscillate a chest of a user. The wearable device includes a chest wall oscillator, a sound detector and a controller for controlling operations of the chest wall oscillator based on sound from the sound detector. The chest wall oscillator may be mounted on the chest of the user to oscillate the chest of the user. The sound detector detects the sound from the chest of the user before, during and/or after operation of the chest wall oscillator. The controller may change one or more of a frequency, intensity or duration of the oscillations of the chest wall oscillator, depending on an analysis of the sound from the sound detector.

US 2018/272147 discloses a wearable system for providing cardiac monitoring, cardiac therapy and/or remote ischemic conditioning therapy.

US 2018/338721 discloses a strap-based wearable device for measuring a physiological parameter of a user.

US 2014/276150 discloses a method for measuring and controlling quality of physiological acoustic signals including lung sounds and heart sounds, and makes use of a pneumatic feedback control system.

SUMMARY OF THE INVENTION

According to the invention, there is provided a wearable device, the wearable device comprising: a controller comprising a processor, an inflatable body configured to be mounted to a torso of a user; a first sensing device, wherein the first sensing device comprises a first sensor and a first actuator coupling the first sensor to the inflatable body; and a memory storing computer-readable instructions that, when executed by the processor, cause the wearable device to: inflate the inflatable body from a first state of the inflatable body to a second state of the inflatable body; actuate the first actuator from a first state of the first actuator to a second state of the first actuator, wherein actuating the first actuator from the first state of the first actuator to the second state of the first actuator reduces a volume of a first air gap between the torso and the first sensor; and while the inflatable body is in the second state of the inflatable body and the first actuator is in the second state of the first actuator, receive a first signal from the first sensor and.

The provision of a wearable device in accordance with the first specific aspect improves the quality of the first signal by allowing the first sensor to be brought into closer proximity with the torso prior to actuation of the first actuator and provides a preliminary amount of air gap reduction that is effective regardless of torso shape.

The wearable device may further comprise a second sensing device. The second sensing device may comprise a second sensor and a second actuator coupling the second sensor to the inflatable body. The computer-readable instructions, when executed, may further cause the wearable device to actuate the second actuator from a first state of the second actuator to a second state of the second actuator and receive a second signal from the second sensor. Actuating the second actuator from the first state of the second actuator to the second state of the second actuator may reduce a volume of a second air gap between the torso and the second sensor. The second signal may be received from the second sensor while the inflatable body is in the second state of the inflatable body and the second actuator is in the second state of the second actuator.

The first sensing device may comprise a first housing that houses the first sensor. The first air gap may be between the torso and the first housing. The first actuator may couple the first housing to the inflatable body and actuating the first actuator from the first state of the first actuator to the second state of the first actuator may reduce the volume of the first air gap between the torso and the first housing. The second sensing device may comprise a second housing that houses the second sensor. The second air gap may be between the torso and the second housing. The second actuator may couple the second housing to the inflatable body and actuating the second actuator from the first state of the second actuator to the second state of the second actuator may reduce the volume of the second air gap between the torso and the second housing.

The first sensor may be a first acoustic sensor. The second sensor may be a second acoustic sensor.

The first actuator may comprise a first inflatable bladder. Actuating the first actuator from the first state of the first actuator to the second state of the first actuator may comprise inflating the first inflatable bladder. The second actuator may comprise a second inflatable bladder. Actuating the second actuator from the first state of the second actuator to the second state of the second actuator may comprise inflating the second inflatable bladder.

The first actuator may comprise a first electroactive polymer. Actuating the first actuator from the first state of the first actuator to the second state of the first actuator may comprise applying a first voltage to the first electroactive polymer. The second actuator may comprise a second electroactive polymer. Actuating the second actuator from the first state of the second actuator to the second state of the second actuator may comprise applying a second voltage to the second electroactive polymer.

According to a second specific aspect, there is provided a method of using a wearable device as described in any preceding statement. The method comprises: inflating the inflatable body from the first state of the inflatable body to the second state of the inflatable body; actuating the first actuator from the first state of the first actuator to the second state of the first actuator, wherein actuating the first actuator from the first state of the first actuator to the second state of the first actuator reduces the volume of the first air gap; and while the inflatable body is in the second state of the inflatable body and the first actuator is in the second state of the first actuator, receiving a first signal from the first sensor.

The provision of a wearable device in accordance with the second specific aspect improves the quality of the first signal by bringing the first sensor into closer proximity with the torso prior to actuation of the first actuator and provides a preliminary amount of air gap reduction that is effective regardless of torso shape.

The method may further comprise actuating the second actuator from the first state of the second actuator to the second state of the second actuator and receiving a second signal from the second sensor. Actuating the second actuator from the first state of the second actuator to the second state of the second actuator may reduce a volume of a second air gap between the torso and the second sensor. The second signal may be received from the second sensor while the inflatable body is in the second state of the inflatable body and the second actuator is in the second state of the second actuator.

The method may further comprise maintaining the first actuator in the second state of the first actuator for a first period of time and receiving the first signal from the first sensor while the first actuator is maintained in the second state of the first actuator. The method may further comprise maintaining the second actuator in the second state of the second actuator for a second period of time and receiving the second signal from the second sensor while the second actuator is maintained in the second state of the second actuator.

The method may further comprise: while the inflatable body is in the second state of the inflatable body, receiving a third signal from the first sensor; determining a first value related to the volume of the first air gap based on the third signal; actuating the first actuator from the first state of the first actuator to the second state of the first actuator based on the first value. The method may further comprise receiving a fourth signal from the second sensor while the inflatable body is in the second state of the inflatable body, determining a second value related to the volume of the second air gap based on the fourth signal and actuating the second actuator from the first state of the second actuator to the second state of the second actuator based on the second value.

The first value may be a signal-to-noise ratio or a signal-to-noise ratio proxy of the third signal. The second value may be a signal-to-noise ratio or a signal-to-noise ratio proxy of the fourth signal.

The method may further comprise the first sensor emitting a first sound pulse and producing the third signal based on a reflection of the first sound pulse. The method may further comprise the second sensor emitting a second sound pulse and producing the fourth signal based on a reflection of the second sound pulse.

The torso comprises a heart. The third signal may be identified as being produced based on a sound emitted by a heartbeat of the heart. The fourth signal may be identified as being produced based on a sound emitted by a heartbeat of the heart.

The torso comprises a lung. The method may further comprise determining a period of time in which the lung is not breathing. The first value may be determined based on the third signal produced during the period of time in which the lung is not breathing. The second value may be determined based on the fourth signal produced during the period of time in which the lung is not breathing.

The method may further comprise actuating the first actuator from the first state of the first actuator to the second state of the first actuator based on a first value. The first value may be determined based on one or more predetermined characteristics of the torso. The method may further comprise actuating the second actuator from the first state of the second actuator to the second state of the second actuator based on a second value. The second value may be determined based on one or more predetermined characteristics of the torso.

These and other aspects will be apparent from and elucidated with reference to the embodiments described hereinafter.

BRIEF DESCRIPTION OF THE DRAWINGS

Exemplary embodiments will now be described, by way of example only, with reference to the following drawings, in which:

Fig. 1 is a schematic cross-sectional view of a wearable device mounted on a torso of a user;
Fig. 2 is a close-up cross-sectional schematic view of the wearable device in a first configuration;
Fig. 3 shows a flow chart of a method of using the wearable device;
Fig. 4 is a close-up cross-sectional schematic view of the wearable device in a second configuration;
Fig. 5 is a graph showing the relationship between air gap volume and signal-to-noise ratio (SNR);
Fig. 6 is a close-up cross-sectional schematic view of the wearable device in a third configuration;
Fig. 7 is a close-up cross-sectional schematic view of a first alternative wearable device in a first configuration;
Fig. 8 is a close-up cross-sectional schematic view of the first alternative wearable device in a second configuration;
Fig. 9 is a close-up cross-sectional schematic view of the first alternative wearable device in a third configuration;
Fig. 10 is a close-up cross-sectional schematic view of a second alternative wearable device in a first configuration;
Fig. 11 is a close-up cross-sectional schematic view of the second alternative wearable device in a second configuration;
Fig. 12 is a close-up cross-sectional schematic view of the second alternative wearable device in a third configuration;
Fig. 13 shows a series of graphs showing the relationship between vest segment compression force or displacement and body shape;
Fig. 14 is a schematic cross-sectional view of a fourth alternative wearable device mounted on a torso of a user;
Fig. 15 is a close-up cross-sectional schematic view of the fourth alternative wearable device in a first configuration;
Fig. 16 shows a flow chart of a method of using the fourth alternative wearable device;
Fig. 17 is a close-up cross-sectional schematic view of the fourth alternative wearable device in a second configuration; and
Fig. 18 is a close-up cross-sectional schematic view of the fourth alternative wearable device in a third configuration.

DETAILED DESCRIPTION OF EMBODIMENTS

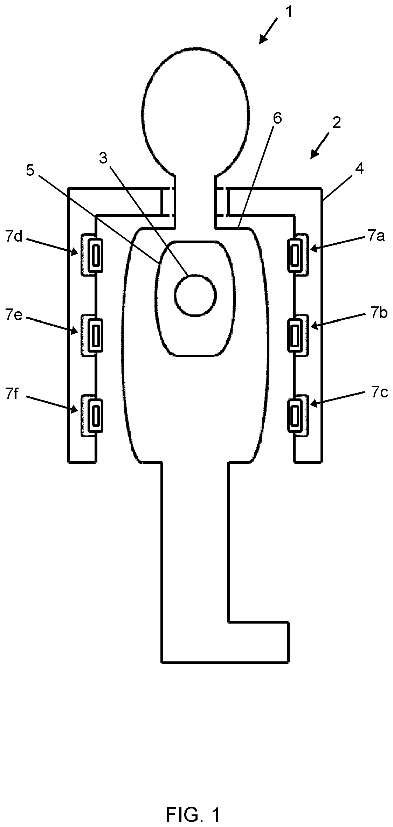

Figure 1 is a schematic cross-sectional view of a wearable device 2 and a user 1. The wearable device 2 comprises an inflatable body 4 which is mounted on a torso 6 of the user 1. The torso 6 comprises a heart 3 and one or more lungs 5. The surface of the torso 6 adjacent the wearable device 2 has a nonplanar surface. The inflatable body 4 is in the form of a vest, such a high-frequency chest wall oscillation (HFCWO) vest. The inflatable body 4 comprises an inflatable body pump 11. The inflatable body pump 11 is an air pump used to drive chest wall oscillation during therapy. The wearable device 2 comprises a plurality of sensing devices 7a-7f. The sensing devices 7a-7f are be located on the wearable device 2 such that they are disposed at positions of interest around the torso 6 (e.g. between ribs, at tracheal positions, at vesicular positions, etc.). The wearable device 2 is shown in a first configuration in Figure 1.

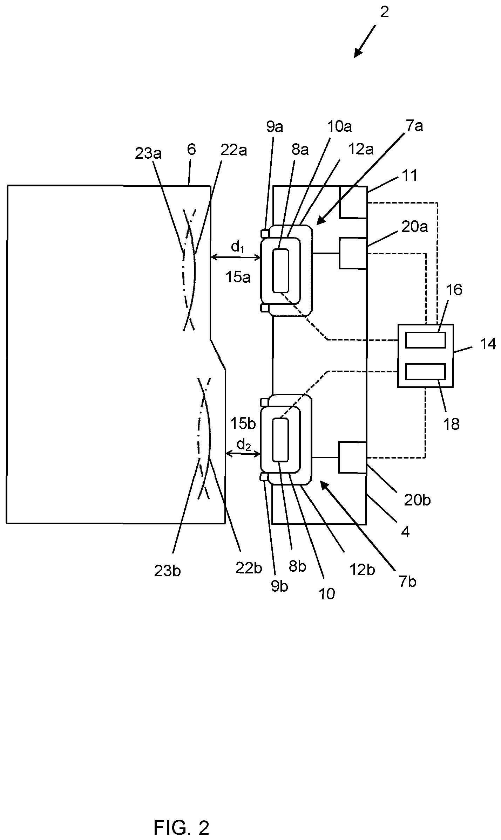

Figure 2 is a close-up cross-sectional schematic view of the wearable device 2 in the first configuration. The inflatable body 4 is in a first, non-inflated state. Figure 2 shows a first sensing device 7a and a second sensing device 7b of the plurality of sensing devices 7a-7f. For clarity, the structure and operation of wearable device 2 and the steps of the associated method 500 in the following description will be described with reference to only the first sensing device 7a and the second sensing device 7b. However, the remaining sensing devices 7c-7f have the same structure and interface with the remaining components of the wearable device 2 in the same manner as the first and second sensing devices 7a, 7b. In addition, the remaining sensing devices 7c-7f operate in the same manner as the first and second sensing devices 7a, 7b. There may be any number of a plurality of sensing devices (i.e. there may be more than six sensing devices).

The first sensing device 7a comprises a first sensor 8a, a first housing 10a housing the first sensor 8a, a first ring of soundproof material 9a and a first actuator 12a. The first actuator 12a couples the first housing 10a to the inflatable body 4, and, thus, couples the first sensor 8a to the inflatable body 4. The first sensor 8a is an acoustic air-coupled sensor in the form of a microphone. The first sensor 8a is configured to produce a series of signals including a first signal and a third signal. The first ring of soundproof material 9a surrounds the first sensor 8a and the first housing 10a. The first actuator 12a comprises a first inflatable bladder. In Figure 2, the first actuator 12a is in a first state, in which the first inflatable bladder is uninflated. A first air gap 15a is disposed between the torso 6 and the first housing 10a, and, thus, between the torso 6 and the first sensor 8a. The first housing 10a is separated from the torso 6 by a first distance di.

The second sensing device 7b comprises a second sensor 8b, a second ring of soundproof material 9a, a second housing 10b housing the second sensor 8b and a second actuator 12b. The second actuator 12b couples the second housing 10b to the inflatable body 4, and, thus couples the second sensor 8b to the inflatable body 4. The second sensor 8b is an acoustic air-coupled sensor in the form of a microphone. The second sensor 8b is configured to produce a series of signals including a second signal and a fourth signal. The second ring of soundproof material 9b surrounds the second sensor 8b and the second housing 10b. The second actuator 12b comprises a second inflatable bladder. In Figure 2, the second actuator 12b is in a first state, in which the second inflatable bladder is uninflated. A second air gap 15b is disposed between the torso 6 and the second housing 10b, and, thus, between the torso 6 and the second sensor 8b. The second housing 10b is separated from the torso 6 by a second distance $d_2$.

The first and second housings 10a, 10b comprise a hydrogel. The hydrogel may be a light-weight hydrogel material. The light-weight hydrogel material may be a cellulose-based hydrogel having a specific acoustic impedance, Z, of between 1.50 and 1.60, a photocrosslinked polyethylene glycol) diacrylate [PEGDA] -based hydrogel having a specific acoustic impedance Z of between 1.53 and 1.66 or silicone rubber (e.g. pure polyurethane rubber having a specific acoustic impedance Z of approximately 1.42. The specific acoustic impedance Z of an adult human chest wall is approximately 1.4 to 1.6 x $10^6$ kg/($m^2$s) based on an average of the acoustic impedance of fat and muscle tissue, which are the two main components of the chest wall.

The first and second air gaps 15a, 15b are formed directly between the torso 6 and the first and second housings 10a, 10b if no other garment (i.e. shirt) is being worn beneath the wearable device 2. If a garment is being worn beneath the wearable device 2, the first and second air gaps 15a, 15b may be formed between the garment and the first and second housings 10a, 10b and/or between the garment and the torso 6.

The wearable device 2 comprises a controller 14. The controller 14 comprises a processor 16 or central processing unit (CPU) and memory 18. The controller 14 is connected to the first and second sensors 8a, 8b. The controller 14 is further connected to a first pump 20a, a second pump 20b and the inflatable body pump 11. The first pump 20a is fluidically connected to the first inflatable bladder 12a. The second pump 20b is fluidically connected to the second inflatable bladder 12b. The memory 18 stores computer-readable instructions that, when executed, cause the device to carry out a method 500.

Soundwaves such as a first soundwave 22a and a second soundwave 22b are generated by the torso 6 and propagate through the torso 6. In the case of measuring lung acoustics related to mucus build-up, the source of the first and second soundwaves 22a, 22b may be lung sounds such as wheezes, crackles and rhonchi which are produced by the lung 5 during inhalation and exhalation by patients with mucus build-up. This sound energy travels from the lung 5 and through the muscle and fat tissue in the chest wall after encountering the interface between the lung 5 and the chest wall, where it is partly reflected.

The specific acoustic impedance Z (characteristic impedance) through a medium is defined by the following equation, in which ρ represents the density of the medium and c represents the speed of sound in the medium:

$$Z = \rho c$$

Impedance mismatch between two adjacent media occurs when the specific acoustic impedance of the two media are different. The greater the difference between the specific acoustic impedances, the higher the level of impedance mismatch.

The amount of sound energy that is reflected in the perpendicular direction from a source as it passes from a first medium with acoustic impedance $Z_1$ to a second medium with acoustic impedance $Z_2$ is referred to as the reflection fraction (RF) or intensity reflection coefficient. The RF between a first medium and a second medium is defined by the following equation, in which $Z_1$ represents the specific acoustic impedance of the first medium and $Z_2$ represents the specific acoustic impedance of the second medium:

$$RF = \frac{(Z_2 - Z_1)^2}{(Z_1 + Z_2)^2}$$

There is a relatively large impedance mismatch between the torso 6 and the first and second air gaps 15a, 15b. Accordingly, a relatively high proportion of the energy transmitted by the first and second soundwaves 22a, 22b is reflected back into the torso 6 at the interface between the torso 6 and the first and second air gaps 15a, 15b as first and second reflections 23a, 23b, and only a relatively small proportion of the energy transmitted by the first and second soundwaves 22a, 22b passes out of the torso 6 and into the first and second air gaps 15a, 15b. There is also a relatively large impedance mismatch between the first and second air gaps 15a, 15b and the first and second housings 10a, 10b. Accordingly, only a relatively small proportion of the energy transmitted from the torso 6 to the first and second air gaps 15a, 15b passes from the first and second air gaps 15a, 15b into the first and second housings 10a, 10b. This results in a relatively small proportion of the acoustic energy present in the first soundwave 22a and second soundwave 22b reaching the first and second sensors 8a, 8b, and, thus, the signals generated by the first and second sensors 8a, 8b have a relatively low SNR and are of poor quality when separated from the torso 6 by a large air gap.

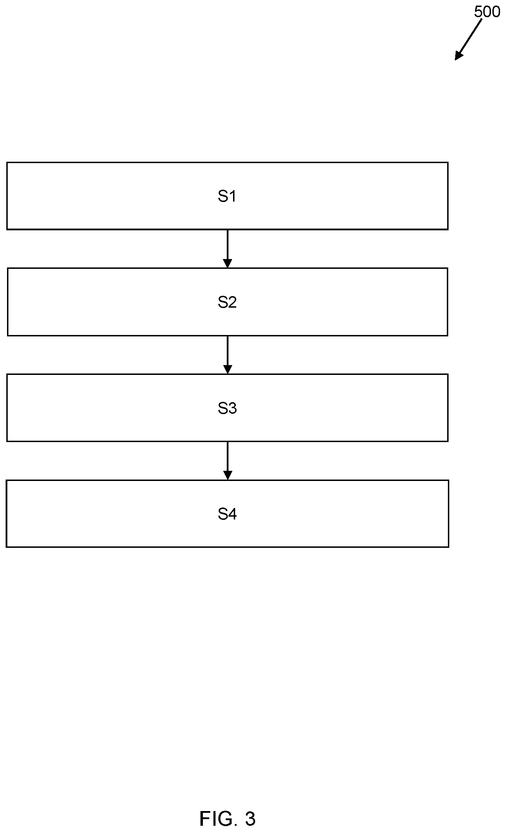

Figure 3 shows a flow chart of the method 500. The method generally comprises a first step S 1, a second step S2, a third step S3 and a fourth step S4. The method may be carried out during a start-up (analytical) mode of the HFCWO vest, to support quantification of mucus volume before beginning therapy. The start-up mode may last between 30 seconds and 120 seconds, for example. The start-up mode can be used to guide and optimize therapy duration, oscillation frequency and displacement. Intermittent analytical checks may be performed during pauses in HFCWO vest therapy (e.g. mucus mobilization) to acquire acoustic measurements to quantify the progress in mucus clearance. This has the advantage of minimizing noise and disturbance of the acoustic measurements.

In the first step S1, the inflatable body 4 is inflated from the first state to a second state using the inflatable body pump 11. When in the second state, the inflatable body 4 is at a base pressure which brings it into close proximity with the torso 6 without being too tight. A pressure sensor (not shown) located within the inflatable body 4 or the inflatable body pump 11 is used to determine the pressure within the inflatable body 4, which is fed back to the controller 14 so that the controller 14 can ensure the inflatable body is inflated to the correct base pressure.

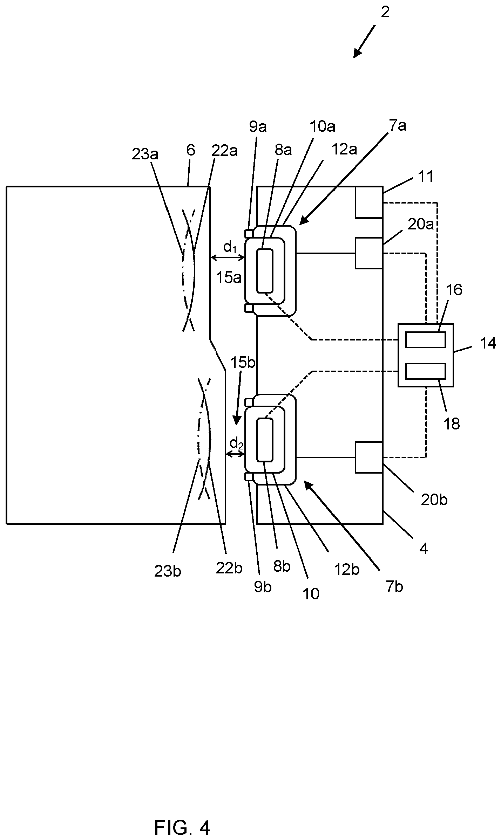

Figure 4 is a close-up cross-sectional schematic view of the wearable device 2 in the second configuration following the first step S1. As shown, the first step S1 reduces the first distance di and reduces the volume of the first air gap 15a between the torso 6 and the first housing 10a, and, thus, between the torso 6 and the first sensor 8a. The first step S1 also reduces the second distance $d_2$ and reduces the volume of the second air gap 15b between the torso 6 and the second housing 10b, and, thus, between the torso 6 and the second sensor 8b.

While the inflatable body 4 is in the second state, the first sensor 8a emits a first sound pulse and the first sensor 8a produces a third signal based on a reflection of the first sound pulse at the interface between the first housing 10a and the first air gap 15a or at the interface between the first housing 10a and the torso 6. The second sensor 8b emits a second sound pulse and the second sensor 8b produces a fourth signal based on a reflection of the second sound pulse at the interface between the second housing 10b and the second air gap 15b or at the interface between the second housing 10b and the torso 6.

A first value related to the volume of the first air gap 15a is determined based on the third signal and a second value related to the volume of the second air gap 15b is determined based on the fourth signal. The first value is an SNR (i.e. the ratio of signal power to noise power) of the third signal and the second value is an SNR of the fourth signal. Noise may be any undesired sounds that interfere with normal and pathologic lung sounds such as heart sounds, clothing friction and movement, motion of the user 1, speaking by the user and background sounds like music or television audio. The device 2 may determine when the lung 5 is not breathing (i.e. between breaths) based on signals outputted by the first and second sensors 8a, 8b, for example. The device 2 may ensure that that the first value is determined based on the third signal produced during the period of time in which the lung 5 is not breathing and ensure that the second value is determined based on the fourth signal produced during the period of time in which the lung 5 is not breathing.

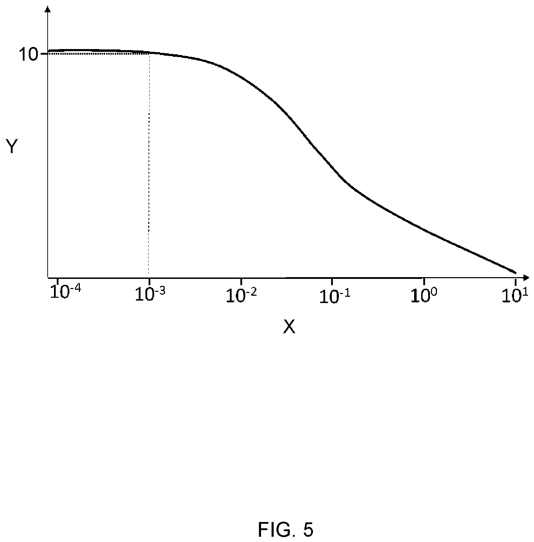

Figure 5 is a graph showing the relationship between the volume of the first and second air gaps 15a, 15b (i.e. the air gap volume) and the SNRs of the third and fourth signals. The air gap volume in millilitres is shown on the x-axis and is denoted by the letter X. The SNR is shown on the y-axis and is denoted by the letter Y. As shown, as the air gap volume decreases, the SNR increases until it reaches a maximum. Once the maximum is reached, the air gap can be considered to be eliminated.

In the second step S2, the first actuator 12a is actuated from the first state to a second state. The first actuator 12a is actuated from the first state to the second state based on the first value. For example, the first actuator 12a may be actuated from the first state to the second state until the first value meets (i.e. exceeds) a threshold value (e.g. an SNR of 10, which, as shown in Figure 5, corresponds to an acceptable air gap volume of $10^{-3}$ ml). Actuating the first actuator 12a from the first state to the second state comprises inflating the first inflatable bladder. When in the second state, the first inflatable bladder 12a is at a pressure that is greater than the base pressure. Actuating the first actuator 12a from the first state to the second state reduces the volume of the first air gap 15a between the torso 6 and the first housing 10a, and, thus, between the torso 6 and the first sensor 8a. The first actuator 12a is maintained in the second state for a first period of time.

In the third step S3, the second actuator 12b is actuated from the first state to a second state. The second actuator 12b is actuated from the first state to the second state based on the second value. For example, the second actuator 12b may be actuated from the first state to the second state until the second value meets (i.e. exceeds) the threshold value (e.g. an SNR of 10, which, as shown in Figure 5, corresponds to an acceptable air gap volume of $10^{-3}$ ml). Actuating the second actuator 12b from the first state to the second state comprises inflating the second inflatable bladder. When in the first state, the second inflatable bladder 12b is at a pressure that is greater than the base pressure. Actuating the second actuator 12b from the first state to the second state reduces the volume of the second air gap 15b between the torso 6 and the second housing 10b, and, thus, between the torso 6 and the second sensor 8b. The second actuator 12b is maintained in the second state for a second period of time. The first and second periods of time are concurrent.

The second and third steps S3, S4 may be repeated multiple times (i.e. iterations) in order to reduce the air gaps to acceptably low volumes.

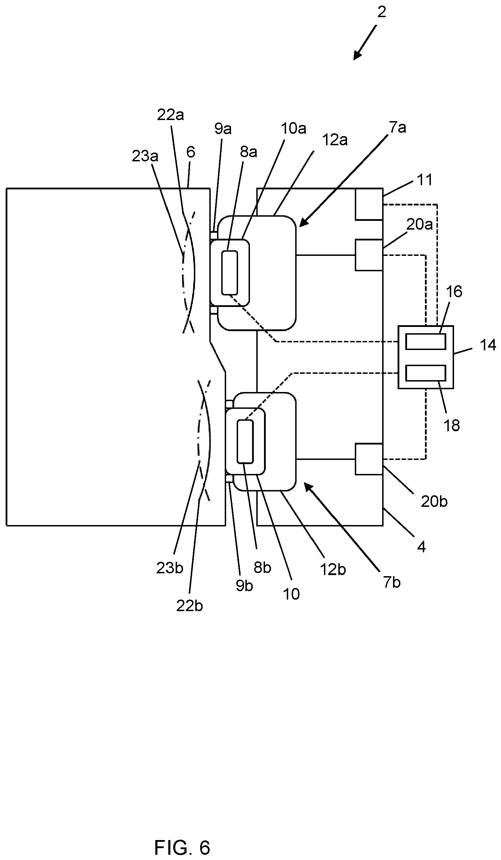

**Figure 6** is a close-up cross-sectional schematic view of the wearable device 2 in the third configuration following the second and third steps S2, S3. As shown, the second step S2 reduces the first distance di and the third step S3 reduces the second distance $d_2$. The first distance di and the second distance $d_2$ may be reduced close to zero. During the second and third steps S2, S3, the first and second actuators 12a, 12b are actuated independently of each other. Accordingly, the first and second actuators 12a, 12b may be actuated by different amounts. For example, in the third configuration shown in Figure 6, the first actuator 12a is actuated by a greater extent that the second actuator 12b, in order to account for the nonplanar surface of the body and to ensure that both actuators 12a, 12b have an acceptably low impedance mismatch.

The second and third steps S2, S3 compress the first and second housings 10a, 10b closely against the torso 6, thereby displacing air trapped between the first and second housings 10a, 10b and the torso 6. A sufficiently large amount of sound energy reaches the first and second housings 10a, 10b from the torso 6 by impedance matching at the interface between the torso 6 and the first and second housings 10a, 10b.

The second and third steps S2, S3 also result in the first and second rings of soundproof material 9a, 9b sealing against the torso 6 such that the amount of background noise reaching the first and second housings 10a, 10b and the first and second sensors 8a, 8b is reduced. In addition or alternatively, the first and second sensors 8a, 8b may be active noise cancelling microphones or be directional microphones directed toward the torso 6.

In the fourth step S4, while the inflatable body 4 is in its second state, the first actuator 12a is in its second state and the second actuator 12b is in its second state, the first sensor 8a produces the first signal and the second sensor 8b produces the second signal. The first signal is produced and received from the first sensor 8a while the first actuator 12a is maintained in the second state, and the second signal is produced and received from the second sensor 8b while the second actuator 12b is maintained in the second state. A first lung function parameter is determined based on the first signal and a second lung function parameter is determined based on the second signal. Pathological lung sounds are typically high frequency lung sounds. Accordingly, the first and second sensors 8a, 8b may be tuned to filter out low frequencies (e.g. frequencies below 1 KHz).

The method 500 ensures reliable and repeatable impedance matching to allow accurate acquisition of lung sounds for acoustic analysis of mucus build-up by improving impedance matching and attenuating noise and acoustic interference during lung sound acquisition, regardless of the shape of the user 1.

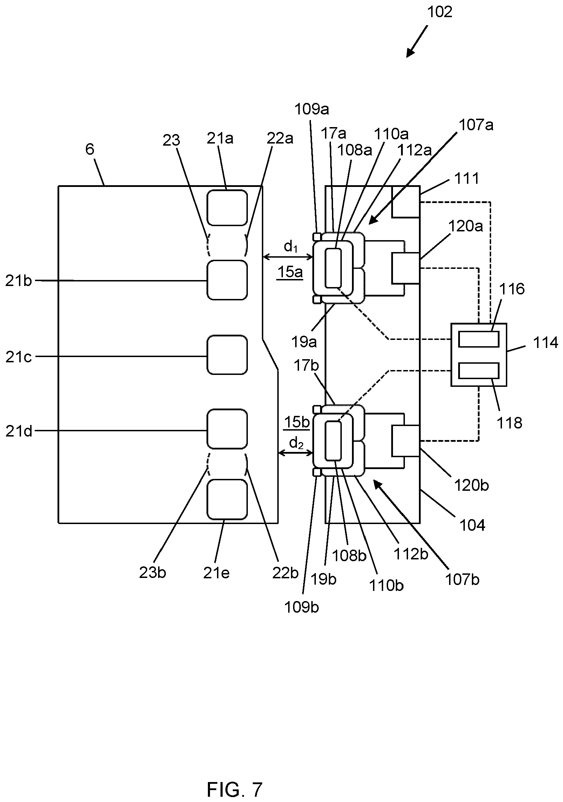

**Figure 7** is a close-up cross-sectional schematic view of a first alternative wearable device 102 in a first configuration. The structure and operation of first alternative wearable device 102 generally corresponds to the structure and operation of the wearable device 2. Corresponding features are denoted using equivalent reference numbers, with the addition of a value of 100.

The first alternative wearable device 102 differs from the wearable device 2 in that the first actuator 112a comprises a first inflatable bladder 17a and a third inflatable bladder 19a and the second actuator 112b comprises a second inflatable bladder 17b and a fourth inflatable bladder 19b. As shown in Figure 7, the torso of the user 1 comprises a first rib 21a, a second rib 21b, a third rib 21c, a fourth rib 21d and a fifth rib 21e.

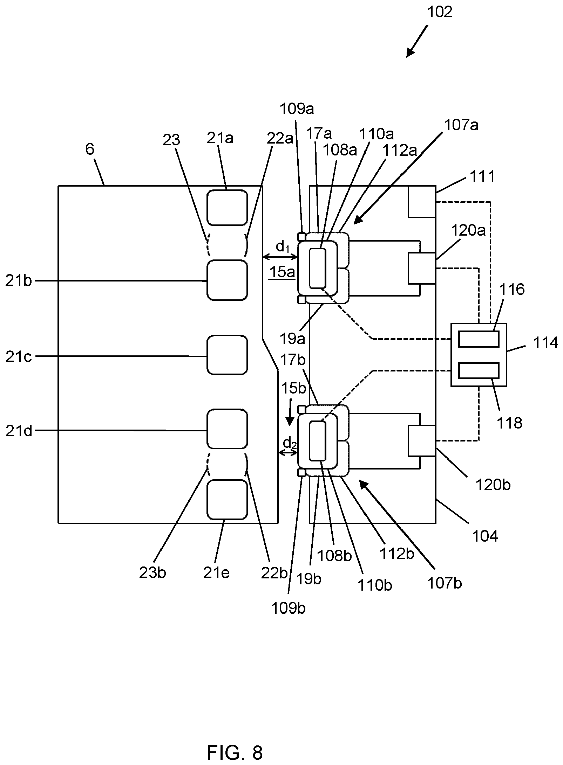

**Figure 8** is a close-up cross-sectional schematic view of the first alternative wearable device 102 in the second configuration following the first step S1. In the second configuration shown in Figure 8, the first soundwave 122a is not aligned with the first sensor 108a and the second soundwave 122b is not aligned with the second sensor 108b. Accordingly, the SNRs of the third and fourth signals are relatively low.

In the second step S2, the first actuator 112a is actuated from the first state to the second state. The first actuator 112a is actuated from the first state to the second state based on the first value, which is an SNR of the third signal. Actuating the first actuator 112a from the first state to the second state comprises inflating the first inflatable bladder 17a and the third inflatable bladder 19a. The first inflatable bladder 17a and the third inflatable bladder 19a may be inflated by different amounts based on the first value. The first value may be obtained while the first housing 110a is in contact with the torso 6. The relative inflation of the first inflatable bladder 17a and the third inflatable bladder 19a may be varied so as to determine the SNR of the third signal for a range of relative inflations and the relative inflation of the first inflatable bladder 17a and the third inflatable bladder 19a that results in the highest (e.g. a peak) SNR of the third signal. The first inflatable bladder 17a and the third inflatable bladder 19a may then be set at the relative inflation that results in the highest SNR of the third signal.

In the third step S3, the second actuator 112b is actuated from the first state to the second state. The second actuator 112b is actuated from the first state to the second state based on the second value, which is an SNR of the fourth signal. Actuating the second actuator 112b from the first state to the second state comprises inflating the second inflatable bladder 17b and the fourth inflatable bladder 19b. The second inflatable bladder 17b and the fourth inflatable bladder 19b may be inflated by different amounts based on the second value. The second value may be obtained while the second housing 110b is in contact with the torso 6. The relative inflation of the second inflatable bladder 17b and the fourth inflatable bladder 19b may be varied so as to determine the SNR of the fourth signal for a range of relative inflations and the relative inflation of the second inflatable bladder 17b and the fourth inflatable bladder 19b that results in the highest (e.g. a peak) SNR of the fourth signal. The second inflatable bladder 17b and the fourth inflatable bladder 19b may then be set at the relative inflation that results in the highest SNR of the fourth signal.

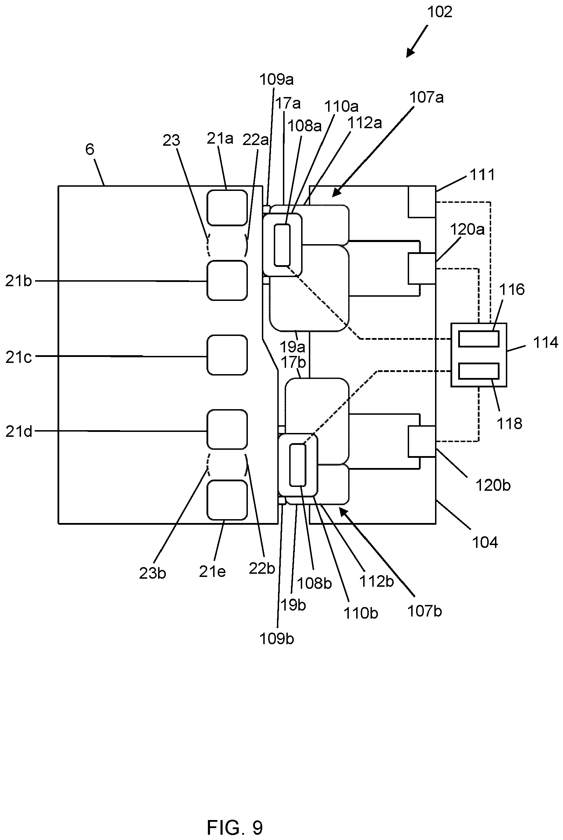

**Figure 9** is a close-up cross-sectional schematic view of the first alternative wearable device 102 in the third configuration following the second and third steps S2, S3. As shown, the second step S2 has resulted in the first sensor 108a being disposed between the first rib 21a and the second rib 21a such that the first sensor 108a is aligned with the first soundwave 122a and has resulted in the second sensor 108b being disposed between the fourth rib 21d and the fifth rib 21e such that the second sensor 108b is aligned with the second soundwave 122b.

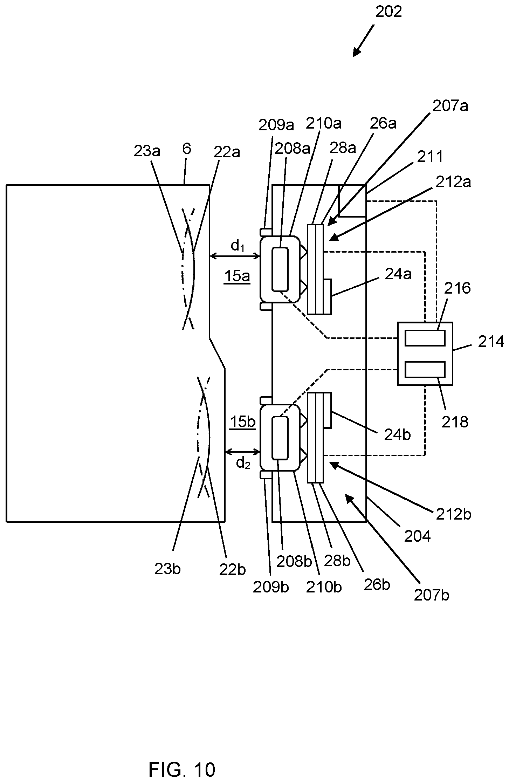

**Figure 10** is a close-up cross-sectional schematic view of a second alternative wearable device 202 in a first configuration. The structure and operation of second alternative wearable device 202 generally corresponds to the structure and operation of the wearable device 2. Corresponding features are denoted using equivalent reference numbers, with the addition of a value of 200.

The second alternative wearable device 202 differs from the wearable device 2 in that the first actuator 212a comprises a first voltage source 24a, a first electroactive polymer (EAP) 26a and a first pressure sensitive element 28a, and the second actuator 212b comprises a second voltage source 24b, a second EAP 26b and a second pressure sensitive element 28b. The first and second pressure sensitive elements 28a, 28b may be first and second pressure sensitive polyvinylidene fluoride (PVDF) foils or sheets. EAPs are lightweight and their displacement and compression force is easily controllable when coupled with a sensing element. The material used for the first and second electroactive polymers (EAP) 26a, 26b may be piezoelectric and electrostrictive polymers, dielectric elastomers, electrostrictive graft polymers, electrostrictive paper, electrets, electroviscoelastic elastomers and liquid crystal elastomers. The EAPs may be field-driven EAPs in which the polymer is sandwiched between two compliant electrodes or in which the EAP is combined with a carrier layer to form a bi-layer configuration. The EAP is stretched (in terms of molecular orientation), which forces the bending in a preferred direction. EAPs can be pre-strained for improved performance in the strained direction (pre-strain leads to better molecular alignment). The electrodes may be metal, since strains usually are in the moderate regime (1-5%). The electrodes may alternatively be formed other materials such as conducting polymers, carbon black based oils, gels, elastomers, etc. The electrodes can be continuous, or segmented.

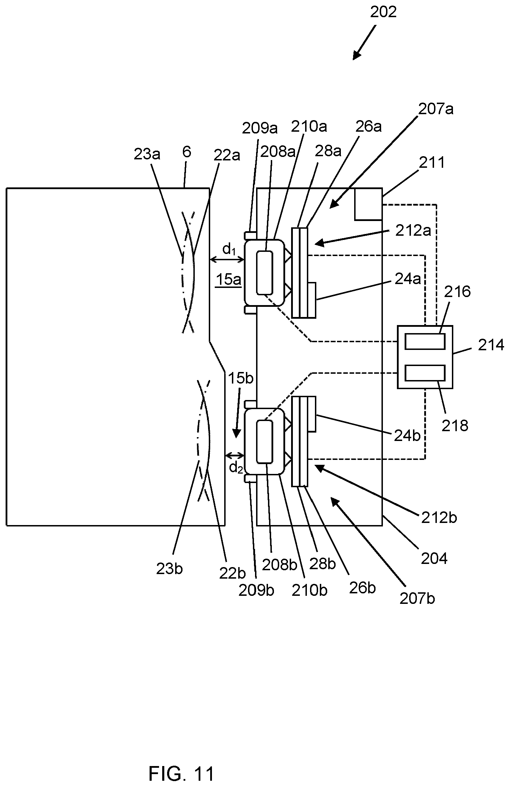

**Figure 11** is a close-up cross-sectional schematic view of the second alternative wearable device 202 in the second configuration following the first step S1.

In the second step S2, actuating the first actuator 212a from the first state to the second state comprises the first voltage source 24a applying a first voltage to the first EAP 26a. This causes the first EAP 26a to expand by a first amount. In the third step S3, actuating the second actuator 212b from the first state to the second state comprises the second voltage source 24b applying a second voltage to the second EAP 26b. This causes the second EAP 26b to expand by a second amount.

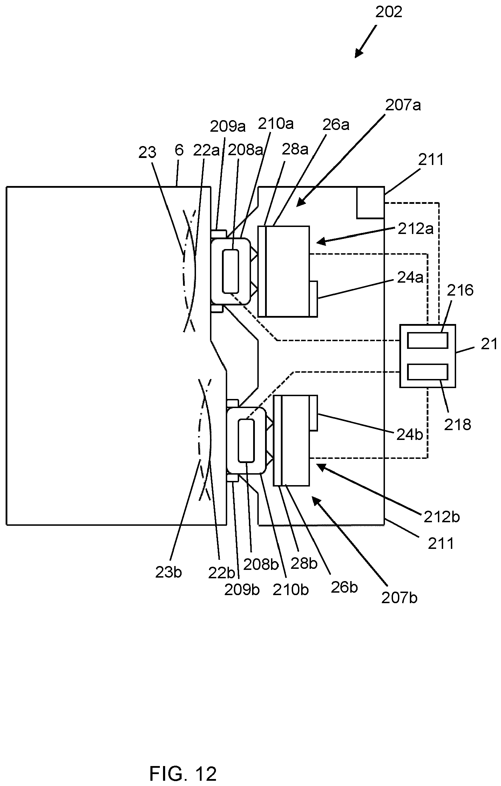

**Figure 12** is a close-up cross-sectional schematic view of the second alternative wearable device 202 in the third configuration following the second and third steps S2, S3.

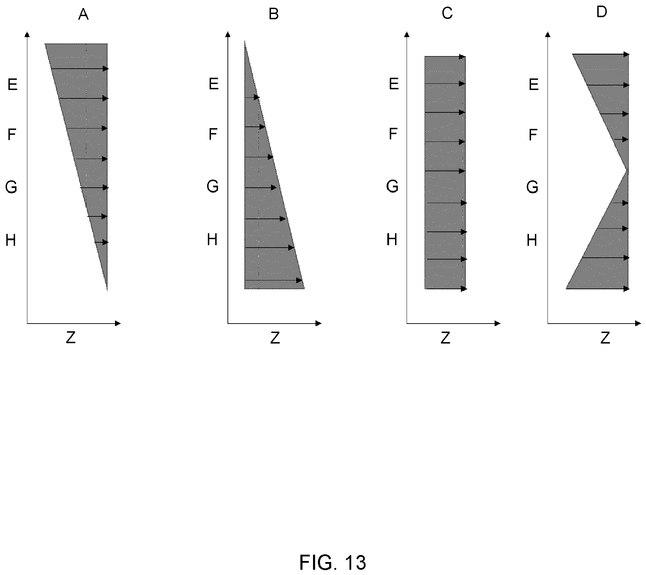

**Figure 13** is a series of graphs showing the relationship between actuation displacement or force on the x-axis (denoted by reference numeral Z) and torso 6 location on the y-axis. Figure 13 demonstrates a method of using a third alternative wearable device. In the third alternative wearable device, the first and second actuators are not actuated based on first and second values that have been determined based on third and fourth signals produced by the first and second sensors. Instead, the first and second values are determined based on one or more predetermined characteristics of the body

By way of a first example of such a third alternative wearable device, an input means may be provided that can be used to input characteristics of the body such as the shape (e.g. morphotype), BMI and/or sex of the torso 6. The values for each of the actuators (i.e. the first and second values, etc.) can be determined based on the inputted characteristics, and the actuators can be actuated based on the values. In Figure 13, the actuator displacement or force for a high BMI (i.e. pyramid) body type across a range of torso 6 locations is shown in graph A, the actuator displacement or force for an inverted pyramid body shape across a range of torso 6 locations is shown in graph B, the actuator displacement or force for a rectangular body shape across a range of torso 6 locations is shown in graph C and the actuator displacement or force for a female body shape across a range of torso 6 locations is shown in graph D.

If the patient has a high BMI (e.g. a BMI greater than or equal to 30), this implies the patient is likely to be wider in the middle torso area than in the upper torso area. Accordingly, the level of actuation of actuators closer to the neck region may be greater than those just above the diaphragm and the level of actuation of the actuators may increase in an upward direction and their values may be set accordingly. If the patient has an inverted pyramidal body shape, the level of actuation of the actuators may increase in a downward direction. If the patient has a flatter, rectangular body shape, the level of actuation of the actuators may be uniform. If the patient has a female body shape, the actuators in the upper and middle chest region may be preferentially actuated by different amounts. For instance, the level of actuation of actuators closer to the patient's clavicle and just above the patient's diaphragm may be greater than the level of actuation of actuators disposed between the patient's clavicle and diaphragm. The level of actuation of the actuators may alternatively be decreased during such a process.

By way of a second example of such an alternative process, scanned geometry of the torso 6 may be obtained using a camera or a 3D laser scanner. The scanned geometry of the torso 6 may be obtained when the patient visits a respiratory therapist or pulmonologist visit in which a HFCWO vest is initially fitted to the patient. The values for each of the actuators (e.g. the first and second values, etc.) can be determined based on the scanned geometry of the torso 6, and the actuators can be actuated based on the values.

The scanned geometry or patient specific data such as BMI, sex and body shape may also be used to set the base pressure of the inflatable body 4. For example, the base pressure for patients with higher BMIs (e.g. greater than or equal to 30) may be set lower than the base pressure for patients with lower BMIs (e.g. below 20). The base pressure can be set using the following scaling law, in which $P_B$ is the optimised base pressure and Po is the standard base pressure:

$$P_B = fP_0 \, , where \, f \propto \frac{1}{BMI}$$

In some arrangements, the first point or points of contact when inflating the inflatable body 4 may be sensed, which can be used to estimate patient body shape and BMI (the first point or points of contact correlate highly with patient body shape and BMI). Subsequently, the actuators can be actuated in an order extending radially outward from the first point or points of contact. In some arrangements, an additional pressure can be created at the back of the sensor using additional actuators to bend it into a convex shape that improves the likelihood of the contact being closer to the centre of the sensor.

In some arrangements, optimized vest inflation settings (e.g. amounts of inflation or actuation) for a particular patient may be stored in memory (e.g. memory 18). The inflation of the inflatable body 4 and the actuation of the actuators may then be set to the optimized vest inflation settings during a subsequent therapy session for the same patient. The optimal settings will likely not change significantly over time as patient body weight and BMI is almost constant, especially over a period of days or weeks. The start-up time of the vest when it is in the mucus build-up quantification mode can therefore be reduced. Optimal vest inflation settings for multiple, different patients (with fairly similar BMI and body shape) can be stored in memory, in order to permit the HFCWO to be shared by multiple users. This could be useful in households with multiple CF or COPD patients or in an out-patient therapy setting.

Although it has been described that the first and second sensors 8a, 8b emit first and second sound pulses and the third and fourth signals are produced by the first and second sensors 8a, 8b based on reflections of the first and second sound pulses, this need not be the case. In alternative arrangements, the first and second sensors 8a, 8b do not emit first and second sound pulses and the third and fourth signals are instead identified as being produced based on a sound emitted by a heartbeat of the heart 3. In such arrangements, the first and second sensors 8a, 8b may be tuned to filter out low frequencies (e.g. such as those produced by heartbeats) only after initial setup of the wearable device 2.

Since the first and second sensors 8a, 8b are placed between the torso 6 and the wearable device 2, they not only pick up sounds produced by the torso 6, but also pick up sounds generated by the wearable device 2 and its inflatable body pump 11. The signal level of the sounds generated by the inflatable body pump 11 is dependent on the on the mechanical contact between the torso 6, the first and second sensors 8a, 8b and the wearable device 2. Accordingly, in addition or alternatively, the third and fourth signals can be identified as being produced based on sounds emitted by the wearable device 2 such as sounds emitted by the inflatable body pump 11.

Although it has been described that the first value is an SNR of the third signal and the second value is an SNR of the fourth signal, this need not be the case. In particular, in alternative arrangements, the first value may be a proxy for SNR of the third signal (e.g. the amplitude of the third signal) and the second value may be an SNR of the fourth signal (e.g. the amplitude of the fourth signal). Several audio signal processing techniques maybe employed to determine the SNR or SNR proxy. For example, the power spectral density (PSD) can be compared between intervals in which the user 1 breathes normally and holds their breath. This allows the differentiation of the actual acoustic signals from the noise floor, and determines the noise and signal power at a given inflation pressure. Alternatively, a matched filter can be applied, which maximizes SNR in the presence of additive stochastic noise (such as that produced when the first and second sensors 8a, 8b emit first and second sound pulses). In this case a template matching approach is used to determine the SNR by comparing the reflected and emitted signal characteristics.

Although it has been described that the first and second sensors 8a, 8b are microphones, they may alternatively be contact sensors such as piezo displacement sensors or accelerometers.

In some arrangements, the wearable device 2 may comprise a plurality of clamping mechanisms. The clamping mechanism may anchor the sensing devices to clothing of the user 1 in order to minimise sensor shift. The sensor shift along the chest may also be measured during or prior to sound acquisition so that the inflation pressure can be adjusted either manually by the patient, or, in alternative arrangements, automatically, to keep the shift below a pre-defined threshold.

Each of the above steps may occur automatically, without the input of the user 1. However, in alternative arrangements, the method of using the wearable device 2 may involve steps that are carried out manually by the user 1. For example, the first and second actuators may be bands or straps and actuating the first and second actuators from the first states to the second states may involve manual actions such as the user 1 tightening the band or straps. After the first step S1 and prior to tightening, the user 1 may be given information relating to the quality of the acoustic signals, and, thus, the size of the air gaps at the sensor locations. The user 1 is then given instructions to tighten the bands or straps near sensors where an air gap has been detected. This can be done by giving the patient an audio-visual report of the locations to be tightened (e.g. on a phone screen, via light indicators on the sensors or a sound indication). Feedback can be provided during the tightening manoeuvre to indicate proper placement, for example via sound signals (e.g. sound tones that increase in frequency based on how close to the optimum tightness the bands or straps have been tightened) or light indicators that change colour.

The contact with torso 6 may be mediated by an air chamber. The air chamber may be enclosed by a diaphragm. In such arrangements, good contact with the torso 6 avoids impedance mismatch or a loss of sensitivity due to a leaking air seal. Microphones having air chambers enclosed by diaphragms have a relatively poor response in the highest relevant frequency range (e.g. 2 to 4 kHz). Accordingly, the inflating pressure may be regulated so as to regulate the tuning of the diaphragm and tune the frequency response of the sensor to a particular frequency range outside this range. In such arrangements, the pressure may be regulated dynamically, for example by changing the pressure range during examination of the torso 6 by altering the inflating pressure after contact with torso 6 has been established.

The first step S1 of the method 500 should precede the second, third and fourth steps S2, S3, S4. Further, the fourth step S4 should follow the second and third steps S3, S4. However, the second step S2 can follow or be carried out at the same time as the third step S3.

As indicated above, the structure and operation of wearable device and the steps of the associated method 500 in the preceding description have been described with reference to only a first sensing device and a second sensing device. However, as indicated above, the wearable device may comprise more than two sensing devices and the remaining sensing devices may have the same structure, interface with the remaining components of the wearable device in the same manner and operate in the same manner as the first and second sensing devices.

The structure and operation of the wearable devices and the steps of the associated method 500 in the preceding description have been described with reference to arrangements comprising multiple sensing devices. However, the wearable device may alternatively comprise a single sensing device, which may have the same structure, interface with the remaining components of the wearable device in the same manner and operate in the same manner as the sensing devices described above.

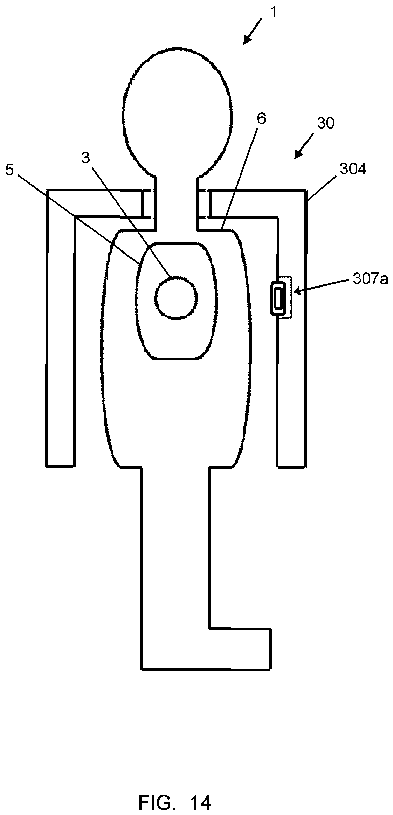

**Figure 14** is a schematic cross-sectional view of a fourth alternative wearable device 302 comprising a single sensing device 307 and a user 1. The structure and operation of the fourth alternative wearable device 302 generally corresponds to the structure and operation of the wearable device 2. Corresponding features are denoted using equivalent reference numbers, with the addition of a value of 300. The wearable device 2 is shown in a first configuration in Figure 14.

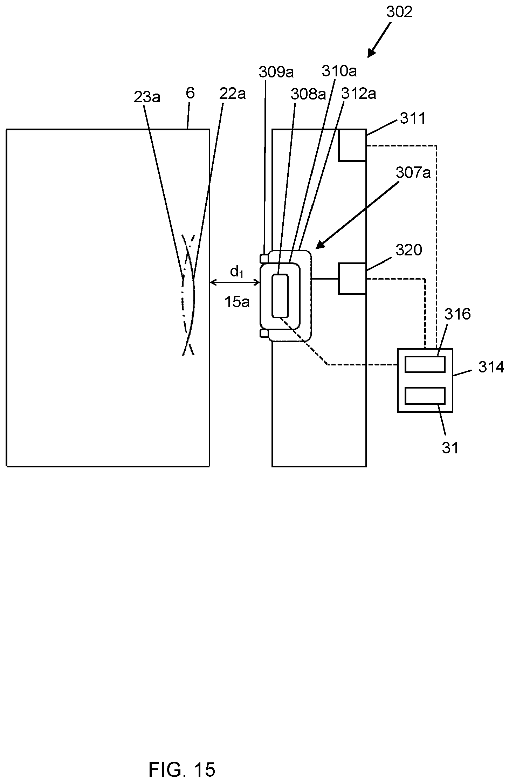

**Figure 15** is a close-up cross-sectional schematic view of the fourth alternative wearable device 302 in the first configuration.

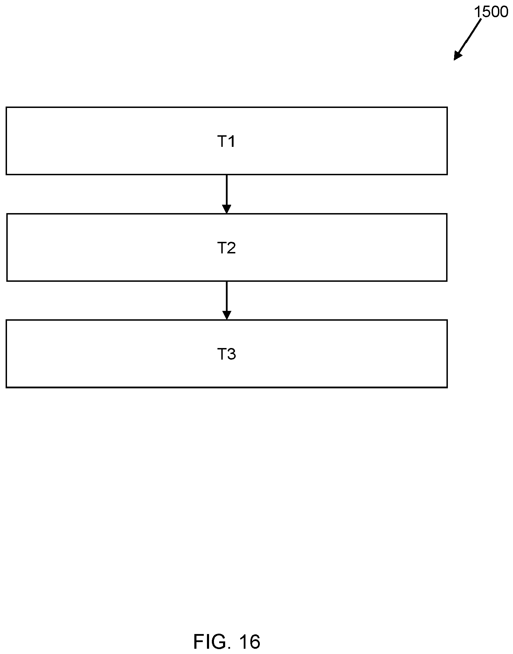

**Figure 16** shows a flow chart of a method 1500 carried out by the fourth alternative wearable device 302. The method 1500 comprises a first step T1, a second step T2 and a third step T3. The first, second and third steps T1, T2, T3 of the method 1500 correspond to the first, second and fourth steps S1, S2, S4 of the method 500 described above, respectively.

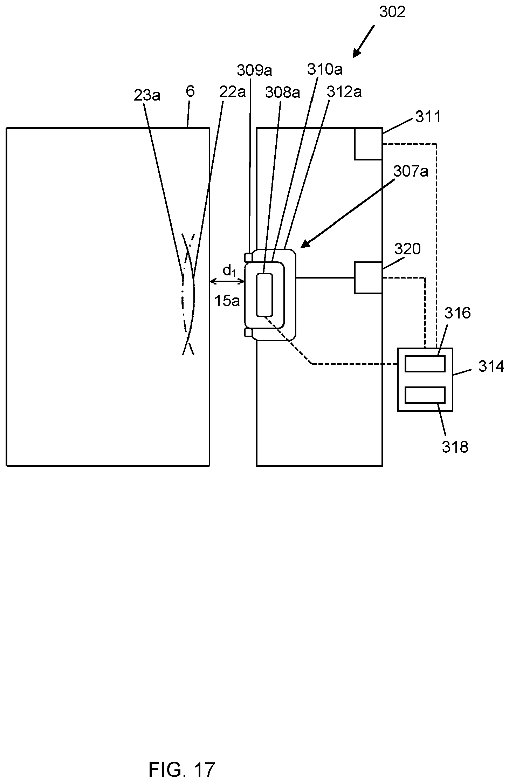

**Figure 17** is a close-up cross-sectional schematic view of the fourth alternative wearable device 302 in the second configuration following the first step T1.

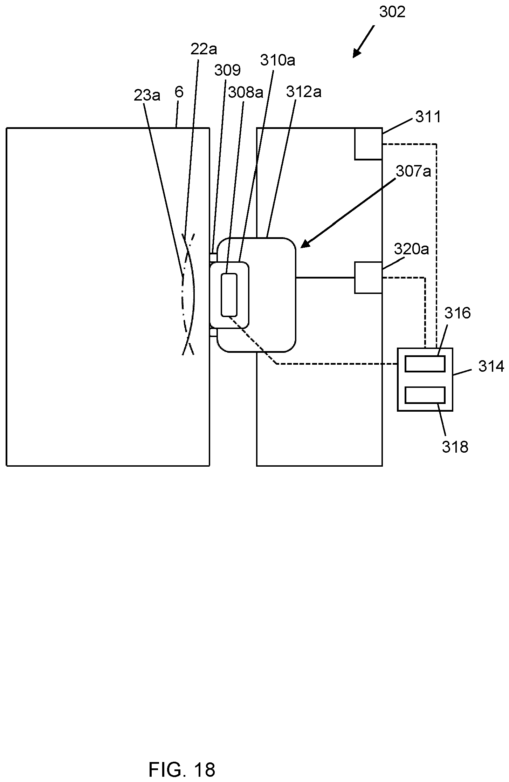

**Figure 18** is a close-up cross-sectional schematic view of the fourth alternative wearable device 302 in the third configuration following the second step T2.

Although the fourth alternative wearable device 302 is shown as comprising a single sensing device corresponding to that described with reference to Figures 1 to 6, the single sensing device may instead correspond to that described with reference to any of the remaining Figures.

For clarity, many feature have been described with reference to a single arrangement. However, the strategies described above may be combined in a single embodiment.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A wearable device (2, 102, 202, 302), the wearable device (2, 102, 202, 302) comprising:

    a controller comprising a processor;
    an inflatable body (4, 104, 204, 304) configured to be mounted to a torso (6) of a user (1);
    a first sensing device (7a, 107a, 207a, 307a), wherein the first sensing device (7a, 107a, 207a, 307a) comprises a first sensor (8a, 108a, 208a, 308a) and a first actuator (12a, 112a, 212a, 312a) coupling the first sensor (8a, 108a, 208a, 308a) to the inflatable body (4, 104, 204, 304); and
    a memory (18, 118, 218, 318) storing computer-readable instructions that, when executed by the processor, cause the wearable device (2, 102, 202, 302) to:

    inflate (S1) the inflatable body (4, 104, 204, 304) from a first state of the inflatable body (4, 104, 204, 304) to a second state of the inflatable body (4, 104, 204, 304);
    actuate (S2) the first actuator (12a, 112a, 212a, 312a) from a first state of the first actuator (12a, 112a, 212a, 312a) to a second state of the first actuator (12a, 112a, 212a, 312a), wherein actuating (S2) the first actuator (12a, 112a, 212a, 312a) from the first state of the first actuator (12a, 112a, 212a, 312a) to the second state of the first actuator (12a, 112a, 212a, 312a) reduces a volume of a first air gap (15a) between the torso (6) and the first sensor (8a, 108a, 208a, 308a); and
    while the inflatable body (4, 104, 204, 304) is in the second state of the inflatable body (4, 104, 204, 304) and the first actuator (12a, 112a, 212a, 312a) is in the second state of the first actuator (12a, 112a, 212a, 312a), receive (S4) a first signal from the first sensor (8a, 108a, 208a, 308a).

2. The wearable device (2, 102, 202) of claim 1, further comprising a second sensing device (7b, 107b, 207b), wherein the second sensing device (7b, 107b, 207b) comprises a second sensor (8b, 108b, 208b) and a second actuator (12b, 112b, 212b) coupling the second sensor (8b, 108b, 208b) to the inflatable body (4, 104, 204), wherein the computer-readable instructions, when executed, further cause the wearable device (2, 102, 202) to actuate (S3) the second actuator (12b, 112b, 212b) from a first state of the second actuator (12b, 112b, 212b) to a second state of the second actuator (12b, 112b, 212b) and receive a second signal from the second sensor (8b, 108b, 208b), wherein actuating (S3) the second actuator (12b, 112b, 212b) from the first state of the second actuator (12b, 112b, 212b) to the second state of the second actuator (12b, 112b, 212b) reduces a volume of a second air gap (15b) between the torso (6) and the second sensor (8b, 108b, 208b), wherein the second signal is received from the second sensor (8b, 108b, 208b) while the inflatable body (4, 104, 204) is in the second state of the inflatable body (4, 104, 204) and the second actuator (12b, 112b, 212b) is in the second state of the second actuator (12b, 112b, 212b).

3. The wearable device (2, 102, 202, 302) of claim 1 or 2, wherein the first sensing device (7a, 107a, 207a, 307a) comprises a first housing (10a, 110a, 210a, 310a) that houses the first sensor (8a, 108a, 208a, 308a) and the first air gap (15a) is between the torso (6) and the first housing (10a, 110a, 210a, 310a), wherein the first actuator (12a, 112a, 212a, 312a) couples the first housing (10a, 110a, 210a, 310a) to the inflatable body (4, 104, 204, 304) and actuating (S2) the first actuator (12a, 112a, 212a, 312a) from the first state of the first actuator (12a, 112a, 212a, 312a) to the second state of the first actuator (12a, 112a, 212a, 312a) reduces the volume of the first air gap (15a) between the torso (6) and the first housing (10a, 110a, 210a, 310a), and wherein optionally the second sensing device (7b, 107b, 207b) comprises a second housing (10b, 110b, 210b) that houses the second sensor (8b, 108b, 208b) and the second air gap (15b) is between the torso (6) and the second housing (10b, 110b, 210b), wherein the second actuator (12b, 112b, 212b) couples the second housing (10b, 110b, 210b) to the inflatable body (4, 104, 204) and actuating (S3) the second actuator (12b, 112b, 212b) from the first state of the second actuator (12b, 112b, 212b) to the second state of the second actuator (12b, 112b, 212b) reduces the volume of the second air gap (15b) between the torso (6) and the second housing (10b, 110b, 210b).

4. The wearable device (2, 102, 202, 302) of any of claims 1 to 3, wherein the first sensor (8a, 108a, 208a, 308a) is a first acoustic sensor and wherein optionally the second sensor (8b, 108b, 208b) is a second acoustic sensor.

5. The wearable device (2, 102, 302) of any of claims 1 to 4, wherein the first actuator (12a, 112a, 312a) comprises a first inflatable bladder, wherein actuating the first actuator (12a, 112a, 312a) from the first state of the first actuator (12a, 112a, 312a) to the second state of the first actuator (12a, 112a, 312a) comprises inflating the first inflatable bladder, wherein optionally the second actuator (12b, 112b) comprises a second inflatable bladder and wherein actuating the second actuator (12b, 112b) from the first state of the second actuator (12b, 112b) to the second state of the second actuator (12b, 112b) comprises inflating the second inflatable bladder.

6. The wearable device (202) of any of claims 1 to 4, wherein the first actuator (212a) comprises a first electroactive polymer (26a), wherein actuating the first actuator (212a) from the first state of the first actuator (212a) to the second state of the first actuator (212a) comprises applying a first voltage to the first electroactive polymer (26a), wherein optionally the second actuator (212b) comprises a second electroactive polymer (26b) and wherein actuating the second actuator (212b) from the first state of the second actuator (212b) to the second state of the second actuator (212b) comprises applying a second voltage to the second electroactive polymer (26b).

7. A method (500, 1500) of using a wearable device (2, 102, 202, 302) as claimed in any of claims 1 to 6, the method (500, 1500) comprising:

inflating (S1) the inflatable body (4, 104, 204, 304) from the first state of the inflatable body (4, 104, 204, 304) to the second state of the inflatable body (4, 104, 204, 304);
actuating (S2) the first actuator (12a, 112a, 212a, 312a) from the first state of the first actuator (12a, 112a, 212a, 312a) to the second state of the first actuator (12a, 112a, 212a, 312a), wherein actuating the first actuator (12a, 112a, 212a, 312a) from the first state of the first actuator (12a, 112a, 212a, 312a) to the second state of the first actuator (12a, 112a, 212a, 312a) reduces the volume of the first air gap (15a); and while the inflatable body (4, 104, 204, 304) is in the second state of the inflatable body (4, 104, 204, 304) and the first actuator (12a, 112a, 212a, 312a) is in the second state of the first actuator (12a, 112a, 212a, 312a), receiving (S4) a first signal from the first sensor (8a, 108a, 208a, 308a).

8. The method (500,1500) of claim 7 when appended to claim 2, further comprising actuating (S3) the second actuator (12b, 112b, 212b) from the first state of the second actuator (12b, 112b, 212b) to the second state of the second actuator (12b, 112b, 212b) and receiving a second signal from the second sensor (8b, 108b, 208b), wherein actuating (S3) the second actuator (12b, 112b, 212b) from the first state of the second actuator (12b, 112b, 212b) to the second state of the second actuator (12b, 112b, 212b) reduces a volume of a second air gap (15b) between the torso (6) and the second sensor (8b, 108b, 208b), wherein the second signal is received from the second sensor (8b, 108b, 208b) while the inflatable body (4, 104, 204) is in the second state of the inflatable body (4, 104, 204) and the second actuator (12b, 112b, 212b) is in the second state of the second actuator (12b, 112b, 212b).

9. The method (500, 1500) of claim 7 or 8, further comprising maintaining the first actuator (12a, 112a, 212a, 312a) in the second state of the first actuator (12a, 112a, 212a, 312a) for a first period of time and receiving the first signal from the first sensor (8a, 108a, 208a, 308a) while the first actuator (12a, 112a, 212a, 312a) is maintained in the second state of the first actuator (12a, 112a, 212a, 312a), wherein the method (500) optionally further comprises maintaining the second actuator (12b, 112b, 212b) in the second state of the second actuator (12b, 112b, 212b) for a second period of time and receiving the second signal from the second sensor (8b, 108b, 208b) while the second actuator (12b, 112b, 212b) is maintained in the second state of the second actuator (12b, 112b, 212b).

10. The method (500, 1500) of any of claims 7 to 9, further comprising:

while the inflatable body (4, 104, 204, 304) is in the second state of the inflatable body (4, 104, 204, 304), receiving a third signal from the first sensor (8a, 108a, 208a, 308a);
determining a first value related to the volume of the first air gap (15a) based on the third signal;
actuating the first actuator (12a, 112a, 212a, 312a) from the first state of the first actuator (12a, 112a, 212a, 312a) to the second state of the first actuator (12a, 112a, 212a, 312a) based on the first value,
wherein optionally the method (500) further comprises receiving a fourth signal from the second sensor (8b, 108b, 208b) while the inflatable body (4, 104, 204) is in the second state of the inflatable body (4, 104, 204), determining a second value related to the volume of the second air gap (15b) based on the fourth signal and actuating the second actuator (12b, 112b, 212b) from the first state of the second actuator (12b, 112b, 212b) to the second state of the second actuator (12b, 112b, 212b) based on the second value.

11. The method (500, 1500) of claim 10, wherein the first value is a signal-to-noise ratio or a signal-to-noise ratio proxy of the third signal and wherein optionally the second value is a signal-to-noise ratio or a signal-to-noise ratio proxy of the fourth signal.

12. The method (500, 1500) of claim 11, further comprising the first sensor (8a, 108a, 208a, 308a) emitting a first sound pulse and producing the third signal based on a reflection of the first sound pulse and wherein optionally the method further comprises the second sensor (8b, 108b, 208b) emitting a second sound pulse and producing the fourth signal based on a reflection of the second sound pulse.

13. The method (500, 1500) of claim 10 or 11, wherein the torso (6) comprises a heart (3) and wherein the third signal is identified as being produced based on a sound emitted by a heartbeat of the heart (3) and wherein optionally the fourth signal is identified as being produced based on a sound emitted by a heartbeat of the heart (3).

14. The method (500, 1500) of any of claims 10 to 13, wherein the torso (6) comprises a lung (5), wherein the method (500, 1500) further comprises determining a period of time in which the lung (5) is not breathing, wherein the first value is determined based on the third signal produced during the period of time in which the lung (5) is not breathing and wherein optionally the second value is determined based on the fourth signal produced during the period of time in which the lung (5) is not breathing.

15. The method (500, 1500) of any of claims 7 to 9, further comprising:

    actuating the first actuator (12a, 112a, 212a, 312a) from the first state of the first actuator (12a, 112a, 212a, 312a) to the second state of the first actuator (12a, 112a, 212a, 312a) based on a first value, wherein the first value is determined based on one or more predetermined characteristics of the torso (6),
    wherein optionally the method (500, 1500) further comprises actuating the second actuator (12b, 112b, 212b) from the first state of the second actuator (12b, 112b, 212b) to the second state of the second actuator (12b, 112b, 212b) based on a second value, wherein the second value is determined based on one or more predetermined characteristics of the torso (6).

## Patentansprüche

1. Tragbare Vorrichtung (2, 102, 202, 302), wobei die tragbare Vorrichtung (2, 102, 202, 302) umfasst:

    eine Steuereinheit, die einen Prozessor umfasst;
    einen aufblasbaren Körper (4, 104, 204, 304), der konfiguriert ist, an einem Rumpf (6) eines Benutzers (1) montiert zu werden;

    eine erste Sensorvorrichtung (7a, 107a, 207a, 307a), wobei die erste Sensorvorrichtung (7a, 107a, 207a, 307a) einen ersten Sensor (8a, 108a, 208a, 308a) und eine erste Betätigungsvorrichtung (12a, 112a, 212a, 312a) umfasst, die den ersten Sensor (8a, 108a, 208a, 308a) mit dem aufblasbaren Körper (4, 104, 204, 304) koppelt; und
    einen Speicher (18, 118, 218, 318), der computerlesbare Anweisungen speichert, die, wenn sie von dem Prozessor ausgeführt werden, die tragbare Vorrichtung (2, 102, 202, 302) veranlassen:

    den aufblasbaren Körper (4, 104, 204, 304) von einem ersten Zustand des aufblasbaren Körpers (4, 104, 204, 304) in einen zweiten Zustand des aufblasbaren Körpers (4, 104, 204, 304) aufzublasen (S1);
    die erste Betätigungsvorrichtung (12a, 112a, 212a, 312a) von einem ersten Zustand der ersten Betätigungsvorrichtung (12a, 112a, 212a, 312a) in einen zweiten Zustand der ersten Betätigungsvorrichtung (12a, 112a, 212a, 312a) zu betätigen (S2), wobei Betätigen (S2) der ersten Betätigungsvorrichtung (12a, 112a, 212a, 312a) von dem ersten Zustand der ersten Betätigungsvorrichtung (12a, 112a, 212a, 312a) in den zweiten Zustand der ersten Betätigungsvorrichtung (12a, 112a, 212a, 312a) ein Volumen eines ersten Luftspalts (15a) zwischen dem Rumpf (6) und dem ersten Sensor (8a, 108a, 208a, 308a) verringert; und
    während sich der aufblasbare Körper (4, 104, 204, 304) in dem zweiten Zustand des aufblasbaren Körpers (4, 104, 204, 304) befindet und sich die erste Betätigungsvorrichtung (12a, 112a, 212a, 312a) in dem zweiten Zustand der ersten Betätigungsvorrichtung (12a, 112a, 212a, 312a) befindet, ein erstes Signal von dem ersten Sensor (8a, 108a, 208a, 308a) zu empfangen (S4).

2. Tragbare Vorrichtung (2, 102, 202) nach Anspruch 1, die weiter eine zweite Sensorvorrichtung (7b, 107b, 207b) umfasst, wobei die zweite Sensorvorrichtung (7b, 107b, 207b) einen zweiten Sensor (8b, 108b, 208b) und eine zweite Betätigungsvorrichtung (12b, 112b, 212b) umfasst, die den zweiten Sensor (8b, 108b, 208b) mit dem aufblasbaren Körper (4, 104, 204) koppelt, wobei die computerlesbaren Anweisungen, wenn ausgeführt, die tragbare Vorrichtung (2, 102, 202) weiter veranlassen, die zweite Betätigungsvorrichtung (12b, 112b, 212b) von einem ersten Zustand der zweiten Betätigungsvorrichtung (12b, 112b, 212b) in einen zweiten Zustand der zweiten Betätigungsvorrichtung (12b, 112b, 212b) zu betätigen (S3) und ein zweites Signal von dem zweiten Sensor (8b, 108b, 208b) zu empfangen, wobei Betätigen (S3) der zweiten Betätigungsvorrichtung (12b, 112b, 212b) von dem ersten Zustand der zweiten Betätigungsvorrichtung (12b, 112b, 212b) in den zweiten Zustand der zweiten Betätigungsvorrichtung (12b, 112b, 212b) ein Volumen eines zweiten Luftspalts (15b) zwischen dem Rumpf (6) und dem zweiten Sensor (8b, 108b, 208b) verringert, wobei das zweite Signal von dem zweiten Sensor (8b, 108b, 208b) empfangen wird, während sich der aufblasbare Körper (4, 104, 204) in dem zweiten Zustand des aufblasbaren Körpers (4, 104, 204) befindet und sich die zweite Betätigungsvorrichtung (12b, 112b, 212b) in dem zweiten Zustand der zweiten Betätigungsvorrichtung (12b, 112b, 212b) befindet.

3. Tragbare Vorrichtung (2, 102, 202, 302) nach Anspruch 1 oder 2, wobei die erste Sensorvorrichtung (7a, 107a, 207a, 307a) ein erstes Gehäuse (10a, 110a, 210a, 310a) umfasst, das den ersten Sensor (8a, 108a, 208a, 308a) beherbergt, und der erste Luftspalt (15a) zwischen dem Rumpf (6) und dem ersten Gehäuse (10a, 110a, 210a, 310a) liegt, wobei die erste Betätigungsvorrichtung (12a, 112a, 212a, 312a) das erste Gehäuse (10a, 110a, 210a, 310a) mit dem aufblasbaren Körper (4, 104, 204, 304) koppelt und Betätigen (S2) der ersten Betätigungsvorrichtung (12a, 112a, 212a, 312a) von dem ersten Zustand der ersten Betätigungsvorrichtung (12a, 112a, 212a, 312a) in den zweiten Zustand der ersten Betätigungsvorrichtung (12a, 112a, 212a, 312a) das Volumen des ersten Luftspalts (15a) zwischen dem Rumpf (6) und dem ersten Gehäuse (10a, 110a, 210a, 310a) verringert, und wobei optional die zweite Sensorvorrichtung (7b, 107b, 207b) ein zweites Gehäuse (10b, 110b, 210b) umfasst, das den zweiten Sensor (8b, 108b, 208b) beherbergt, und der zweite Luftspalt (15b) zwischen dem Rumpf (6) und dem zweiten Gehäuse (10b, 110b, 210b) liegt, wobei die zweite Betätigungsvorrichtung (12b, 112b, 212b) das zweite Gehäuse (10b, 110b, 210b) mit dem aufblasbaren Körper (4, 104, 204) koppelt und Betätigen (S3) der zweiten Betätigungsvorrichtung (12b, 112b, 212b) von dem ersten Zustand der zweiten Betätigungsvorrichtung (12b, 112b, 212b) in den zweiten Zustand der zweiten Betätigungsvorrichtung (12b, 112b, 212b) das Volumen das zweiten Luftspalts (15b) zwischen dem Rumpf (6) und dem zweiten Gehäuse (10b, 110b, 210b) verringert.

4. Tragbare Vorrichtung (2, 102, 202, 302) nach einem der Ansprüche 1 bis 3, wobei der erste Sensor (8a, 108a, 208a, 308a) ein erster akustischer Sensor ist und wobei optional der zweite Sensor (8b, 108b, 208b) ein zweiter akustischer Sensor ist.

5. Tragbare Vorrichtung (2, 102, 302) nach einem der Ansprüche 1 bis 4, wobei die erste Betätigungsvorrichtung (12a, 112a, 312a) eine erste aufblasbare Blase umfasst, wobei Betätigen der ersten Betätigungsvorrichtung (12a, 112a, 312a) von dem ersten Zustand der ersten Betätigungsvorrichtung (12a, 112a, 312a) in den zweiten Zustand der ersten Betätigungsvorrichtung (12a, 112a, 312a) Aufblasen der ersten aufblasbaren Blase umfasst, wobei optional die zweite Betätigungsvorrichtung (12b, 112b) eine zweite aufblasbare Blase umfasst und wobei Betätigen der zweiten Betätigungsvorrichtung (12b, 112b) von dem ersten Zustand der zweiten Betätigungsvorrichtung (12b, 112b) in den zweiten Zustand der zweiten Betätigungsvorrichtung (12b, 112b) Aufblasen der zweiten aufblasbaren Blase umfasst.

6. Tragbare Vorrichtung (202) nach einem der Ansprüche 1 bis 4, wobei die erste Betätigungsvorrichtung (212a) ein erstes elektroaktives Polymer (26a) umfasst, wobei Betätigen der ersten Betätigungsvorrichtung (212a) von dem ersten Zustand der ersten Betätigungsvorrichtung (212a) in den zweiten Zustand der ersten Betätigungsvorrichtung (212a) Anlegen einer ersten Spannung an das erste elektroaktive Polymer (26a) umfasst, wobei optional die zweite Betätigungsvorrichtung (212b) ein zweites elektroaktives Polymer (26b) umfasst und wobei Betätigen der zweiten Betätigungsvorrichtung (212b) von dem ersten Zustand der zweiten Betätigungsvorrichtung (212b) in den zweiten Zustand der zweiten Betätigungsvorrichtung (212b) Anlegen einer zweiten Spannung an das zweite elektroaktive Polymer (26b) umfasst.

7. Verfahren (500, 1500) zum Verwenden einer tragbaren Vorrichtung (2, 102, 202, 302) nach einem der Ansprüche 1 bis 6, wobei das Verfahren (500, 1500) umfasst:

Aufblasen (S1) des aufblasbaren Körpers (4, 104, 204, 304) von dem ersten Zustand des aufblasbaren Körpers (4, 104, 204, 304) in den zweiten Zustand des aufblasbaren Körpers (4, 104, 204, 304);
Betätigen (S2) der ersten Betätigungsvorrichtung (12a, 112a, 212a, 312a) von dem ersten Zustand der ersten Betätigungsvorrichtung (12a, 112a, 212a, 312a) in den zweiten Zustand der ersten Betätigungsvorrichtung (12a, 112a, 212a, 312a), wobei Betätigen der ersten Betätigungsvorrichtung (12a, 112a, 212a, 312a) von dem ersten Zustand der ersten Betätigungsvorrichtung (12a, 112a, 212a, 312a) in den zweiten Zustand der ersten Betätigungsvorrichtung (12a, 112a, 212a, 312a) das Volumen des ersten Luftspalts (15a) verringert; und

während sich der aufblasbare Körper (4, 104, 204, 304) in dem zweiten Zustand des aufblasbaren Körpers (4, 104, 204, 304) befindet und sich die erste Betätigungsvorrichtung (12a, 112a, 212a, 312a) in dem zweiten Zustand der ersten Betätigungsvorrichtung (12a, 112a, 212a, 312a) befindet, Empfangen (S4) eines ersten Signals von dem ersten Sensor (8a, 108a, 208a, 308a).

8. Verfahren (500, 1500) nach Anspruch 7, wenn an Anspruch 2 angehängt, das weiter Betätigen (S3) der zweiten Betätigungsvorrichtung (12b, 112b, 212b) von dem ersten Zustand der zweiten Betätigungsvorrichtung (12b, 112b, 212b) in den zweiten Zustand der zweiten Betätigungsvorrichtung (12b, 112b, 212b) und Empfangen eines zweiten Signals von dem zweiten Sensor (8b, 108b, 208b) umfasst, wobei Betätigen (S3) der zweiten Betätigungsvorrichtung (12b, 112b, 212b) von dem ersten Zustand der zweiten Betätigungsvorrichtung (12b, 112b, 212b) in den zweiten Zustand der zweiten Betätigungsvorrichtung (12b, 112b, 212b) ein Volumen eines zweiten Luftspalts (15b) zwischen dem Rumpf (6) und dem zweiten Sensor (8b, 108b, 208b) verringert, wobei das zweite Signal von dem zweiten Sensor (8b, 108b, 208b) empfangen wird, während sich der aufblasbare Körper (4, 104, 204) in dem zweiten Zustand des aufblasbaren Körpers (4, 104, 204) befindet und sich die zweite Betätigungsvorrichtung (12b, 112b, 212b) in dem zweiten Zustand der zweiten Betätigungsvorrichtung (12b, 112b, 212b) befindet.

9. Verfahren (500, 1500) nach Anspruch 7 oder 8, das weiter Halten der ersten Betätigungsvorrichtung (12a, 112a, 212a, 312a) in dem zweiten Zustand der ersten Betätigungsvorrichtung (12a, 112a, 212a, 312a) eine erste Zeitspanne lange, und Empfangen des ersten Signals von dem ersten Sensor (8a, 108a, 208a, 308a) umfasst, während die erste Betätigungsvorrichtung (12a, 112a, 212a, 312a) in dem zweiten Zustand der ersten Betätigungsvorrichtung (12a, 112a, 212a, 312a) gehalten wird, wobei das Verfahren (500) optional weiter Halten der zweiten Betätigungsvorrichtung (12b, 112b, 212b) in dem zweiten Zustand der zweiten Betätigungsvorrichtung (12b, 112b, 212b) eine zweite Zeitspanne lang, und Empfangen des zweiten Signals von dem zweiten Sensor (8b, 108b, 208b) umfasst, während die zweite Betätigungsvorrichtung (12b, 112b, 212b) in dem zweiten Zustand der zweiten Betätigungsvorrichtung (12b, 112b, 212b) gehalten wird.

10. Verfahren (500, 1500) nach einem der Ansprüche 7 bis 9, weiter umfassend:

während sich der aufblasbare Körper (4, 104, 204, 304) in dem zweiten Zustand des aufblasbaren Körpers (4, 104, 204, 304) befindet, Empfangen eines dritten Signals von dem ersten Sensor (8a, 108a, 208a, 308a);
Bestimmen eines ersten Werts in Bezug auf das Volumen des ersten Luftspalts (15a) basierend auf dem dritten Signal;
Betätigen der ersten Betätigungsvorrichtung (12a, 112a, 212a, 312a) von dem ersten Zustand der ersten Betätigungsvorrichtung (12a, 112a, 212a, 312a) in den zweiten Zustand der ersten Betätigungsvorrichtung (12a, 112a, 212a, 312a) basierend auf dem ersten Wert, wobei optional das Verfahren (500) weiter Empfangen eines vierten Signals von dem zweiten Sensor (8b, 108b, 208b), während sich der aufblasbare Körper (4, 104, 204) in dem zweiten Zustand des aufblasbaren Körpers (4,104,204) befindet, Bestimmen eines zweiten Werts in Bezug auf das Volumen des zweiten Luftspalts (15b) basierend auf dem vierten Signal, und Betätigen der zweiten Betätigungsvorrichtung (12b, 112b, 212b) von dem ersten Zustand der zweiten Betätigungsvorrichtung (12b, 112b, 212b) in den zweiten Zustand der zweiten Betätigungsvorrichtung (12b, 112b, 212b) basierend auf dem zweiten Wert umfasst.

11. Verfahren (500, 1500) nach Anspruch 10, wobei der erste Wert ein Signal-Rausch-Verhältnis oder ein Signal-Rausch-Verhältnis-Proxy des dritten Signals ist, und wobei optional der zweite Wert ein Signal-Rausch-Verhältnis oder ein Signal-Rausch-Verhältnis-Proxy des vierten Signals ist.

12. Verfahren (500, 1500) nach Anspruch 11, das weiter umfasst, dass der erste Sensor (8a, 108a, 208a, 308a) einen ersten Schallimpuls aussendet und das dritte Signal basierend auf einer Reflexion des ersten Schallimpulses erzeugt, und wobei optional das Verfahren weiter umfasst, dass der zweite Sensor (8b, 108b, 208b) einen zweiten Schallimpuls aussendet und das vierte Signal basierend auf einer Reflexion des zweiten Schallimpulses erzeugt.

13. Verfahren (500, 1500) nach Anspruch 10 oder 11, wobei der Rumpf (6) ein Herz (3) umfasst und wobei das dritte Signal als basierend auf einem durch einen Herzschlag des Herzens (3) ausgesendeten Schall erzeugt identifiziert wird und wobei optional das vierte Signal als basierend auf einem durch einen Herzschlag des Herzens (3) ausgesendeten Schall erzeugt identifiziert wird.

14. Verfahren (500, 1500) nach einem der Ansprüche 10 bis 13, wobei der Rumpf (6) eine Lunge (5) umfasst, wobei das Verfahren (500, 1500) weiter Bestimmen eines Zeitraums umfasst, in dem die Lunge (5) nicht atmet, wobei der erste Wert basierend auf dem dritten Signal bestimmt wird, das während des Zeitraums erzeugt wird, in dem die Lunge (5) nicht atmet, und wobei optional der zweite Wert basierend auf dem vierten Signal bestimmt wird, das während des Zeitraums erzeugt wird, in dem die Lunge (5) nicht atmet.

15. Verfahren (500, 1500) nach einem der Ansprüche 7 bis 9, weiter umfassend:

Betätigen der ersten Betätigungsvorrichtung (12a, 112a, 212a, 312a) von dem ersten Zustand der ersten Betätigungsvorrichtung (12a, 112a, 212a, 312a) in den zweiten Zustand der ersten Betätigungsvorrichtung (12a, 112a, 212a, 312a) basierend auf einem ersten Wert, wobei der erste Wert basierend auf einem oder mehreren vorbestimmten Eigenschaften des Rumpfes (6) bestimmt wird, wobei optional das Verfahren (500, 1500) weiter Betätigen der zweiten Betätigungsvorrichtung (12b, 112b, 212b) von dem ersten Zustand der zweiten Betätigungsvorrichtung (12b, 112b, 212b) in den zweiten Zustand der zweiten Betätigungsvorrichtung (12b, 112b, 212b) basierend auf einem zweiten Wert umfasst, wobei der zweite Wert basierend auf einem oder mehreren vorbestimmten Eigenschaften des Rumpfes (6) bestimmt wird.

## Revendications

1. Dispositif portable (2, 102, 202, 302), le dispositif portable (2, 102, 202, 302) comprenant :

un dispositif de commande comprenant un processeur ;
un corps gonflable (4, 104, 204, 304) configuré pour être installé sur un torse (6) d'un utilisateur (1) ;
un premier dispositif de détection (7a, 107a, 207a, 307a), dans lequel le premier dispositif de détection (7a, 107a, 207a, 307a) comprend un premier capteur (8a, 108a, 208a, 308a) et un premier actionneur (12a, 112a, 212a, 312a) couplant le premier capteur (8a, 108a, 208a, 308a) au corps gonflable (4, 104, 204, 304) ; et
une mémoire (18, 118, 218, 318) stockant des instructions lisibles par ordinateur qui, lorsqu'elles sont exécutées par le processeur, amènent le dispositif portable (2, 102, 202, 302) à :

gonfler (51) le corps gonflable (4, 104, 204, 304) d'un premier état du corps gonflable (4, 104, 204, 304) à un second état du corps gonflable (4, 104, 204, 304) ;

actionner (S2) le premier actionneur (12a, 112a, 212a, 312a) d'un premier état du premier actionneur (12a, 112a, 212a, 312a) à un second état du premier actionneur (12a, 112a, 212a, 312a), dans lequel l'actionnement (S2) du premier actionneur (12a, 112a, 212a, 312a) du premier état du premier actionneur (12a, 112a, 212a, 312a) au second état du premier actionneur (12a, 112a, 212a, 312a) réduit un volume d'un premier espace d'air (15a) entre le torse (6) et le premier capteur (8a, 108a, 208a, 308a) ; et
en même temps que le corps gonflable (4, 104, 204, 304) est dans le second état du corps gonflable (4, 104, 204, 304) et le premier actionneur (12a, 112a, 212a, 312a) est dans le second état du premier actionneur (12a, 112a, 212a, 312a), recevoir (S4) un premier signal provenant du premier capteur (8a, 108a, 208a, 308a).

2. Dispositif portable (2, 102, 202) selon la revendication 1, comprenant en outre un second dispositif de détection (7b, 107b, 207b), dans lequel le second dispositif de détection (7b, 107b, 207b) comprend un second capteur (8b, 108b, 208b) et un second actionneur (12b, 112b, 212b) couplant le second capteur (8b, 108b, 208b) au corps gonflable (4, 104, 204), dans lequel les instructions lisibles par ordinateur, lorsqu'elles sont exécutées, amènent en outre le dispositif portable (2, 102, 202) à actionner (S3) le second actionneur (12b, 112b, 212b) d'un premier état du second actionneur (12b, 112b, 212b) à un second état du second actionneur (12b, 112b, 212b) et recevoir un deuxième signal en provenance du second capteur (8b, 108b, 208b), dans lequel l'actionnement (S3) du second actionneur (12b, 112b, 212b) du premier état du second actionneur (12b, 112b, 212b) au second état du second actionneur (12b, 112b, 212b) réduit un volume d'un second espace d'air (15b) entre le torse (6) et le second capteur (8b, 108b, 208b), dans lequel le deuxième signal est reçu en provenance du second capteur (8b, 108b, 208b) en même temps que le corps gonflable (4, 104, 204) est dans le second état du corps gonflable (4, 104, 204) et le second actionneur (12b, 112b, 212b) est dans le second état du second actionneur (12b, 112b, 212b).

3. Dispositif portable (2, 102, 202, 302) selon la revendication 1 ou 2, dans lequel le premier dispositif de détection (7a, 107a, 207a, 307a) comprend un premier boîtier (10a, 110a, 210a, 310a) qui loge le premier capteur (8a, 108a, 208a, 308a) et le premier espace d'air (15a) se trouve entre le torse (6) et le premier boîtier (10a, 110a, 210a, 310a), dans lequel le premier actionneur (12a, 112a, 212a, 312a) couple le premier boîtier (10a, 110a, 210a, 310a) au corps gonflable (4, 104, 204, 304) et l'actionnement (S2) du premier actionneur (12a, 112a, 212a, 312a) du premier état du premier actionneur (12a, 112a, 212a, 312a) au second état du premier actionneur (12a, 112a, 212a, 312a) réduit le volume du premier espace d'air (15a) entre le torse (6) et le premier boîtier (10a, 110a, 210a, 310a), et dans lequel facultativement le second dispositif de détection (7b, 107b, 207b) comprend un second boîtier (10b, 110b, 210b) qui loge le second capteur (8b, 108b, 208b) et le second espace d'air (15b) se trouve entre le torse (6) et le second boîtier (10b, 110b, 210b), dans lequel le second actionneur (12b, 112b, 212b) couple le second boîtier (10b, 110b, 210b) au corps gonflable (4, 104, 204) et l'actionnement (S3) du second actionneur (12b, 112b, 212b) du premier état du second actionneur (12b, 112b, 212b) au second état du second actionneur (12b, 112b, 212b) réduit le volume du second espace d'air (15b) entre le torse (6) et le second boîtier (10b, 110b, 210b).loge

4. Dispositif portable (2, 102, 202, 302) selon l'une quelconque des revendications 1 à 3, dans lequel le premier capteur (8a, 108a, 208a, 308a) est un premier capteur acoustique et dans lequel facultativement le second capteur (8b, 108b, 208b) est un second capteur acoustique.

5. Dispositif portable (2, 102, 302) selon l'une quelconque des revendications 1 à 4, dans lequel le premier actionneur (12a, 112a, 312a) comprend une première vessie gonflable, dans lequel l'actionnement du premier actionneur (12a, 112a, 312a) du premier état du premier actionneur (12a, 112a, 312a) au second état du premier actionneur (12a, 112a, 312a) comprend le gonflage de la première vessie gonflable, dans lequel facultativement le second actionneur (12b, 112b) comprend une seconde vessie gonflable et dans lequel l'actionnement du second actionneur (12b, 112b) du premier état du second actionneur (12b, 112b) au second état du second actionneur (12b, 112b) comprend le gonflage de la seconde vessie gonflable.

6. Dispositif portable (202) selon l'une quelconque des revendications 1 à 4, dans lequel le premier actionneur (212a) comprend un premier polymère électroactif (26a), dans lequel l'actionnement du premier actionneur (212a) du premier état du premier actionneur (212a) au second état du premier actionneur (212a) comprend l'application d'une première tension au premier polymère électroactif (26a), dans lequel facultativement le second actionneur (212b) comprend un second polymère électroactif (26b) et dans lequel l'actionnement du second actionneur (212b) du premier état du second actionneur (212b) au second état du second actionneur (212b) comprend l'application d'une seconde tension au second polymère électroactif (26b).

7. Procédé (500, 1500) d'utilisation d'un dispositif portable (2, 102, 202, 302) selon l'une quelconque des revendications 1 à 6, le procédé (500, 1500) comprenant :

le gonflage (S1) du corps gonflable (4, 104, 204, 304) du premier état du corps gonflable (4, 104, 204, 304) au second état du corps gonflable (4, 104, 204, 304) ;
l'actionnement (S2) du premier actionneur (12a, 112a, 212a, 312a) du premier état du premier actionneur (12a, 112a, 212a, 312a) au second état du premier actionneur (12a, 112a, 212a, 312a), dans lequel l'actionnement du premier actionneur (12a, 112a, 212a, 312a) du premier état du premier actionneur (12a, 112a, 212a, 312a) au second état du premier actionneur (12a, 112a, 212a, 312a) réduit le volume du premier espace d'air (15a) ; et
en même temps que le corps gonflable (4, 104, 204, 304) est dans le second état du corps gonflable (4, 104, 204, 304) et le premier actionneur (12a, 112a, 212a, 312a) est dans le second état du premier actionneur (12a, 112a, 212a, 312a), la réception (S4) d'un premier signal provenant du premier capteur (8a, 108a, 208a, 308a).

8. Procédé (500, 1500) selon la revendication 7 lorsqu'elle est annexée à la revendication 2, comprenant en outre l'actionnement (S3) du second actionneur (12b, 112b, 212b) du premier état du second actionneur (12b, 112b, 212b) au second état du second actionneur (12b, 112b, 212b) et la réception d'un deuxième signal provenant du second capteur (8b, 108b, 208b), dans lequel l'actionnement (S3) du second actionneur (12b, 112b, 212b) du premier état du second actionneur (12b, 112b, 212b) au second état du second actionneur (12b, 112b, 212b) réduit un volume d'un second espace d'air (15b) entre le torse (6) et le second capteur (8b, 108b, 208b), dans lequel le deuxième signal est reçu en provenance du second capteur (8b, 108b, 208b) en même temps que le corps gonflable (4, 104, 204) est dans le second état du corps gonflable (4, 104, 204) et le second actionneur (12b, 112b, 212b) est dans le second état du second actionneur (12b, 112b, 212b).

9. Procédé (500, 1500) selon la revendication 7 ou 8, comprenant en outre le maintien du premier actionneur (12a, 112a, 212a, 312a) dans le second état du premier actionneur (12a, 112a, 212a, 312a) pendant une première période de temps et la réception du premier signal provenant du premier capteur (8a, 108a, 208a, 308a) en même temps que le premier actionneur (12a, 112a, 212a, 312a) est maintenu dans le second état du premier actionneur (12a, 112a, 212a, 312a), dans lequel le procédé (500) comprend en outre facultativement le maintien du second actionneur (12b, 112b, 212b) dans le second état du second actionneur (12b, 112b, 212b) pendant une seconde période de temps et la réception du deuxième signal provenant du second capteur (8b, 108b, 208b) en même temps que le second actionneur (12b, 112b, 212b) est maintenu dans le second état du second actionneur (12b, 112b, 212b).

10. Procédé (500, 1500) selon l'une quelconque des revendications 7 à 9, comprenant en outre :

en même temps que le corps gonflable (4, 104, 204, 304) est dans le second état du corps gonflable (4, 104, 204, 304), la réception d'un troisième signal en provenance du premier capteur (8a, 108a, 208a, 308a) ;
la détermination d'une première valeur liée au volume du premier espace d'air (15a) sur la base du troisième signal ;
l'actionnement du premier actionneur (12a, 112a, 212a, 312a) du premier état du premier actionneur (12a, 112a, 212a, 312a) au second état du premier actionneur (12a, 112a, 212a, 312a) sur la base de la première valeur,
dans lequel facultativement le procédé (500) comprend en outre la réception d'un quatrième signal provenant du second capteur (8b, 108b, 208b) en même temps que le corps gonflable (4, 104, 204) est dans le second état du corps gonflable (4, 104, 204), la détermination d'une seconde valeur liée au volume du second espace d'air (15b) sur la base du quatrième signal et l'actionnement du second actionneur (12b, 112b, 212b) du premier état du second actionneur (12b, 112b, 212b) au second état du second actionneur (12b, 112b, 212b) sur la base de la seconde valeur.

11. Procédé (500, 1500) selon la revendication 10, dans lequel la première valeur est un rapport de signal sur bruit ou une approximation de rapport de signal sur bruit du troisième signal et dans lequel facultativement la seconde valeur est un rapport de signal sur bruit ou une approximation de rapport de signal sur bruit du quatrième signal.

12. Procédé (500, 1500) selon la revendication 11, comprenant en outre le premier capteur (8a, 108a, 208a, 308a) émettant une première impulsion sonore et produisant le troisième signal sur la base d'une réflexion de la première impulsion sonore et dans lequel facultativement le procédé comprend en outre le second capteur (8b, 108b, 208b) émettant une seconde impulsion sonore et produisant le quatrième signal sur la base d'une réflexion de la seconde impulsion sonore.

13. Procédé (500, 1500) selon la revendication 10 ou 11, dans lequel le torse (6) comprend un coeur (3) et dans lequel le troisième signal est identifié comme étant produit sur la base d'un son émis par un battement de coeur du coeur (3) et dans lequel facultativement le quatrième signal est identifié comme étant produit sur la base d'un son émis par un battement de coeur du coeur (3).

14. Procédé (500, 1500) selon l'une quelconque des revendications 10 à 13, dans lequel le torse (6) comprend un poumon (5), dans lequel le procédé (500, 1500) comprend en outre la détermination d'une période de temps pendant laquelle le poumon (5) ne respire pas, dans lequel la première valeur est déterminée sur la base du troisième signal produit pendant la période de temps pendant laquelle le poumon (5) ne respire pas et dans lequel facultativement la seconde valeur est déterminée sur la base du quatrième signal produit pendant la période de temps pendant laquelle le poumon (5) ne respire pas.

15. Procédé (500, 1500) selon l'une quelconque des revendications 7 à 9, comprenant en outre :

l'actionnement du premier actionneur (12a, 112a, 212a, 312a) du premier état du premier actionneur (12a, 112a, 212a, 312a) au second état du premier actionneur (12a, 112a, 212a, 312a) sur la base d'une première valeur, dans lequel la première valeur est déterminée sur la base d'une ou plusieurs caractéristiques prédéterminées du torse (6),
dans lequel facultativement le procédé (500, 1500) comprend en outre l'actionnement du second actionneur (12b, 112b, 212b) du premier état du second actionneur (12b, 112b, 212b) au second état du second actionneur (12b, 112b, 212b) sur la base d'une seconde valeur, dans lequel la seconde valeur est déterminée sur la base d'une ou plusieurs caractéristiques prédéterminées du torse (6).

FIG. 1

FIG. 2

500

FIG. 3

FIG. 4

FIG. 5

FIG. 6

FIG. 7

EP 4 213 738 B1

FIG. 8

26

FIG. 9

FIG. 10

FIG. 11

FIG. 12

FIG. 13

FIG. 14

FIG. 15

1500

```
┌─────────────────────────────────────────────┐
│                                             │
│                    T1                       │
│                                             │
└─────────────────────────────────────────────┘
                       │
                       ▼
┌─────────────────────────────────────────────┐
│                                             │
│                    T2                       │
│                                             │
└─────────────────────────────────────────────┘
                       │
                       ▼
┌─────────────────────────────────────────────┐
│                                             │
│                    T3                       │
│                                             │
└─────────────────────────────────────────────┘
```

FIG. 16

FIG. 17

FIG. 18

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20190142686 A
- US 2018272147 A
- US 2018338721 A
- US 2014276150 A